# EUROPÄISCHE PATENTSCHRIFT

(11) **EP 2 623 274 B1**
(45) Veröffentlichungstag und Bekanntmachung des Hinweises auf die Patenterteilung: **03.04.2019**
(21) Anmeldenummer: 12190320.7
(22) Anmeldetag: 29.10.2012
(51) Int. Cl.: B25J 9/16, A61B 1/00, A61B 1/04, B25J 19/00, B25J 19/06

(54) **Magnetischer Endeffektor und Einrichtung zur Führung und Positionierung desselben**
Magnetic end effector and device for guiding and positioning the same
Effecteur terminal magnétique et dispositif de guidage et de positionnement de celui-ci

(30) Priorität: 28.10.2011 DE 102011054910
(43) Veröffentlichungstag der Anmeldung: 07.08.2013
(60) Teilanmeldung: 15188357.6
(73) Patentinhaber: Ovesco Endoscopy AG, 72074 Tübingen (DE)
(72) Erfinder: Dr. Schostek, Sebastian, 72074 Tübingen (DE); Gottwald, Thomas, Prof., 82431 Kochel am See (DE); Schurr, Marc O., Dr., 72072 Tübingen (DE)
(74) Vertreter: Winter, Brandl, Fürniss, Hübner, Röss, Kaiser, Polte - Partnerschaft mbB

(56) Entgegenhaltungen:
- EP-A1- 0 554 711
- EP-A1- 2 347 699
- WO-A1-03/099152
- WO-A1-2005/122866
- WO-A1-2009/107892
- DE-B3- 10 335 644
- KR-B1- 100 735 863
- GASTONE CIUTIA ET AL: "Robotic magnetic steering and locomotion of capsule endoscope for diagnostic and surgical endoluminal procedures", ROBOTICA, CAMBRIDGE, GB, Bd. 28, 1. Januar 2010 (2010-01-01), Seiten 199-207, XP008119581, ISSN: 0263-5747, DOI: 10.1017/S0263574709990361 [gefunden am 2009-10-26]
- THOMAS W R FOUNTAIN ET AL: "Wireless control of magnetic helical microrobots using a rotating-permanent-magnet manipulator", 2010 IEEE INTERNATIONAL CONFERENCE ON ROBOTICS AND AUTOMATION : ICRA 2010 ; ANCHORAGE, ALASKA, USA, 3 - 8 MAY 2010, IEEE, PISCATAWAY, NJ, USA, 3. Mai 2010 (2010-05-03), Seiten 576-581, XP031743182, ISBN: 978-1-4244-5038-1

## Beschreibung

Die vorliegende Erfindung betrifft allgemein ein medizinisches Robotersystem zur magnetischen Führung einer Sonde und insbesondere eine magnetische Führungseinrichtung für ein intrakorporales Objekt, vorzugsweise eine Endoskopkapsel oder ein Katheter. Die erfindungsgemäße Führungseinrichtung wird nachfolgend am Beispiel der Anwendung in der Medizin beschrieben. Die Erfindung lässt sich vollumfänglich aber auch auf andere technische Systeme übertragen. Ein solches technisches System kann beispielsweise ein Rohrleitungssystem sein, in dem sich das zu steuernde Objekt befindet.

### Hintergrund der Erfindung

Benachbarter Stand der Technik ist auch aus den Druckschriften WO 2005/122866 A1 und EP 2 347 699 A1 bekannt. So offenbart die erstgenannte Veröffentlichung ein kapselartiges Endoskop-Kontrollsystem. Die zweitgenannte Druckschrift offenbart ein ähnliches System mit einem Magnetantrieb. Ein ähnliches Endoskopsystem ist auch aus der WO 2009/107892 A1 bekannt. In dem Zusammenhang ist die Patentschrift KR 100 735 863 B1 auch von Interesse. Die Veröffentlichung von Gastone Ciutia et al. mit dem Titel "Robotic magnetic steering and locomotion of capsule endoscope for diagnostic and surgical endoluminal procedures" sowie die Veröffentlichung von Thomas W. R. Fountain et al. mit dem Titel "Wireless control of magnetic helical microrobots using a rotating-permanent-magnet manipulator" mit der ISBN-Nummer 978-1-4244-5038-1 ist ebenfalls erwähnenswert.

Benachbarter Stand der Technik ist auch aus den Druckschriften DE 103 35 644 B3, EP 0 554 711 A1 und WO 03/099152 A1 bekannt.

In der Medizin sind Systeme (Robotersysteme) zur (manuellen oder vorprogrammierten) Steuerung intrakorporaler Objekte mittels extrakorporal erzeugter Magnetfelder bekannt. Intrakorporale Objekte, zum Beispiel endoskopische Kapseln, Sonden oder Katheter, weisen hierzu ein durch Magnetfelder beeinflussbares Element auf, beispielsweise einen Permanentmagneten, der in dem Objekt verbaut ist. Durch extrakorporal erzeugte Magnetfelder lassen sich die Ausrichtung und / oder Positionierung der intrakorporalen Objekte steuern. Zur Bereitstellung extrakorporaler Magnetfelder sind ebenfalls Systeme zur Erzeugung elektromagnetischer Gradientenfelder, beispielsweise nach Art von Magnetresonanztomographen, elektromagnetischer Punktfelder, beispielsweise durch einen Elektromagneten in kompakter Bauweise, sowie Systeme mit Permanentmagnet bekannt.

Systeme zur Erzeugung elektromagnetischer Gradientenfelder, beispielsweise nach Bauart von Magnetresonanztomographen, haben den Nachteil, dass diese Geräte im Vergleich zu in der medizinischen Praxis oder Klinik sonst üblichen elektrischen Verbrauchern einen sehr hohen Energiebedarf aufweisen und durch die Bauart bedingt im Vergleich zu in der medizinischen Praxis oder Klinik sonst üblichen technischen Geräten sehr groß und schwer sind. Vorteile der Systeme nach Bauart von

Magnetresonanztomographen sind indessen, dass das Gradientenfeld am Ort der intrakorporalen Objekte zur Steuerung derselben fein justiert und reproduzierbar eingestellt werden kann, und dass eine Patientengefährdung durch bewegliche Bauteile weitgehend ausgeschlossen werden kann, da sich die beweglichen Teile innerhalb einer den Patienten statisch umgebenden rigiden Verschalung befinden.

Unter dem Begriff "Magnetfelderzeuger kompakter Bauart" sollen nachfolgend solche Vorrichtungen verstanden werden, die ein Magnetfeld erzeugen, das sich im Raum um die Vorrichtung herum erstreckt, so dass sich die Vorrichtung im Zentrum des erzeugten Magnetfeldes befindet. Insbesondere erstreckt sich der Nutzbereich jener Magnetfelder, die von Magnetfelderzeugern kompakter Bauart erzeugt werden, im räumlichen Umfeld dieser Magnetfelderzeuger. Im Gegensatz dazu stehen Erzeuger eines Gradientenfeldes, beispielsweise nach Bauart eines Magnetresonanztomographen, dessen Nutzbereich sich im Inneren einer ringförmigen Vorrichtung erstreckt, in die ein Patient eingeschoben werden muss, d.h. das nutzbare Magnetfeld soll den Magnetfelderzeuger nicht umgeben sondern sich in eine Richtung (radial einwärts der ringförmigen Vorrichtung) von diesem weg erstrecken. Magnetfelderzeuger kompakter Bauart können beispielsweise aus einem oder mehrerer Elektromagneten oder einem oder mehrerer Permanentmagneten oder Kombinationen beider bestehen.

Der Begriff "Kraft", wie er nachfolgend allgemein verwendet wird, kann eine mechanische Kraft oder ein Drehmoment beschreiben.

Die Verwendung von Magnetfelderzeugern kompakter Bauart gemäß vorstehender Definition hat den Vorteil, dass die Geräte im Vergleich zu in der medizinischen Praxis und Klinik sonst üblichen elektrischen Verbrauchern einen ähnlichen Energiebedarf aufweisen, und durch die Bauart bedingt im Vergleich zu in der medizinischen Praxis oder Klinik sonst üblichen technischen Geräten eine ähnliche Größe und Gewicht besitzen. D.h. sie sind auch für den mobilen Einsatz in einer Praxis oder Klinik mit üblichem Aufwand prinzipiell geeignet. Ein Nachteil der Verwendung von Magnetfelderzeugern kompakter Bauart zur Steuerung eines intrakorporalen Objektes ist jedoch, dass zur Einstellung des
Magnetfeldes am Ort des intrakorporalen Objektes die Lage und Ausrichtung des Magnetfelderzeugers kompakter Bauart (z.B. Permanentmagnet oder Elektromagnet) im Raum erforderlich ist, d.h. der Magnetfelderzeuger kompakter Bauart muss bezüglich eines Patienten im Raum bewegt werden. Die Ausrichtung des Magnetfelderzeugers kompakter Bauart im Raum wird nach heutigem Stand der Technik entweder manuell oder durch aktuierte (motorisch angetriebene), robotische Vorrichtungen, d.h. sogenannte magnetische Führungseinrichtungen erreicht. Ein weiterer Nachteil von Magnetfelderzeugern kompakter Bauart ist, dass sich die Stärke des Magnetfeldes im Nutzbereich mit steigendem Abstand zum Magnetfelderzeuger stark reduziert. Aus diesem Grund ist es vorteilhaft und ggf. auch notwendig, die Magnetfelderzeuger kompakter Bauart so nah wie möglich an das zu steuernde intrakorporale Objekt zu positionieren, so dass eine für die Steuerung des intrakorporalen Objektes ausreichender Einfluss des Magnetfeldes auf das intrakorporale Objekt gegeben ist.

Die manuell geführte Ausrichtung des Magnetfelderzeugers kompakter Bauart hat den Vorteil, dass der Aufbau eine minimale Komplexität aufweisen kann. Ausführungsbeispiele umfassen die Bewegung eines in der Hand gehaltenen Permanentmagneten sowie handgeführte, passive, d.h. nicht aktuierte Unterstützungssysteme zur Gewichtskompensation des Magnetfelderzeugers kompakter Bauart. Ein weiterer Vorteil ist, dass der Steuernde durch die manuelle Ausrichtung des Magnetfelderzeugers kompakter Bauart über zumindest seinen haptischen Sinn eine permanente Informationsrückkopplung über die Bewegungsparameter und insbesondere Kollisionen mit und Krafteinwirkung auf den Patientenkörper erhält, und damit den Patienten gefährdende Einwirkungen bei der Ausrichtung des Magnetfelderzeugers kompakter Bauart vermieden werden können. Die manuell geführte Ausrichtung des Magnetfelderzeugers kompakter Bauart im Raum hat jedoch den Nachteil, dass der Steuerungseffekt von Ausrichtungsbewegungen des Magnetfelderzeugers kompakter Bauart auf die Ausrichtung des intrakorporalen Objektes nicht intuitiv vorhersagbar ist, da der Steuernde keine direkte Sichtverbindung zu dem intrakorporalen Objekt hat und damit auch keine Informationen zu dessen aktueller Position und Ausrichtung erhält. Das heißt, dem Steuernden können keine unmittelbare Informationen über die aktuelle Position und Ausrichtung des intrakorporalen Objekts über den Magnetfelderzeuger vermittelt werden, was beispielsweise in der Fliegerei eigentlich einem Nachtflug auf Sicht entspräche. Es ist klar, dass ein Führungsverfahren auf der Basis der vorstehend beschriebenen Gerätschaften nur mit sehr viel Übung und voraussichtlich mit unzureichenden Resultaten durchführbar ist. Dieser Umstand ist beispielsweise in der EP 2 347 699 A1 näher beschrieben.

Die robotisch geführte Ausrichtung des Magnetfelderzeugers kompakter Bauart im Raum hat dem gegenüber den Vorteil, dass durch eine direkte Informationsrückkopplung zwischen dem intrakorporalen Objekt und der den Magnetfelderzeuger kompakter Bauart steuernden robotischen Vorrichtung (Roboterarm) eine für den Steuernden intuitive und vorhersagbare Steuerung von Position und Ausrichtung des intrakorporalen Objekts erreicht werden kann. Das Prinzip einer solchen Informationsrückkopplung ist in der vorstehend genannten EP 2 347 699 A1 offenbart, sodass zum vollständigen Verständnis der vorliegenden technischen Lehre an dieser Stelle auf die entsprechende Druckschrift verwiesen werden kann.

Eine robotisch geführte Ausrichtung des Magnetfelderzeugers kompakter Bauart im Raum hat allerdings den Nachteil, dass die Vorrichtung zur robotisch geführten Ausrichtung des Magnetfelderzeugers kompakter Bauart auch solche Bewegungen grundsätzlich tätigen kann, die ggf. zu einer Kollision mit und ohne Krafteinwirkung auf den Patientenkörper und damit zu den Patienten oder den Steuernden gefährdenden Einwirkungen auf dessen Körper führen können. In anderen Worten ausgedrückt sind Roboter konventioneller Konzeption ohne weiteres dazu in der Lage, bei einer falschen Bewegung einen Menschen tödlich zu verletzen oder umstehende Gegenstände zu beschädigen, sodass normalerweise ein Sicherheitsbereich im Bewegungsfeld des betreffenden Roboters abgesperrt werden muss.

Wie bereits vorstehend ausgeführt wurde, ist es vorteilhaft, den Magnetfelderzeuger kompakter Bauart möglichst nahe an das zu steuernde intrakorporale Objekt zu positionieren, um eine ausreichende magnetische Anziehungskraft auf das Objekt auszuüben. Es kann sogar von Vorteil sein, den Magnetfelderzeuger kompakter Bauart mit einer definierten Kraft auf den Patientenkörper zu drücken, um eine Eindellung des Körpers beispielsweise im Bauchbereich zu erzeugen, die eine Positionierung des Magnetfelderzeugers kompakter Bauart noch näher an das zu steuernde intrakorporale Objekt ermöglicht. In einer solchen Situation können sich Bewegungen des Magnetfelderzeugers sowohl zur Positionierung als auch Ausrichtung des Magnetfelderzeugers kompakter Bauart auf den Patientenkörper unmittelbar (mechanisch) übertragen und zu den Patienten gefährdenden Einwirkungen auf den Patientenkörper führen. Es ist auch offensichtlich, dass Sicherheitsbereiche in herkömmlichen Sinn in diesem Fall nicht einsetzbar sind.

Es ist technisch möglich, so genannte virtuelle Barrieren zu etablieren, um Kollisionen bzw. übergebührliche Krafteinwirkungen auf den Patientenkörper zu vermeiden. Virtuelle Barrieren sind in die Software des Steuerungssystems der Vorrichtung zur robotisch geführten Ausrichtung des Magnetfelderzeugers kompakter Bauart in Form von Raumkoordinaten oder Aktuatorpositionen hinterlegte Grenzwerte, die bei bestimmungsgemäßer Funktion der Vorrichtung nicht überschritten werden, d.h. es besteht prinzipiell die Möglichkeit, den vorstehenden Sicherheitsbereich virtuell über programmtechnische Maßnahmen zu errichten. Nachteil dieser technischen Maßnahme ist jedoch, dass dies nicht die individuellen anatomischen Gegebenheiten von Patienten (dick, schlank, Mann, Frau, etc) berücksichtigen kann. Somit ist eine bestmögliche Annäherung des Magnetfelderzeugers kompakter Bauart an das zu steuernde intrakorporale Objekt nicht mehr in jedem Fall gegeben. Ein weiterer Nachteil von virtuellen Barrieren ist, dass diese auf Softwareebene implementiert sind und somit zur Erkennung von Fehlfunktionen weitere Maßnahmen auf Softwareebene getroffen werden müssen. Fehlfunktionen können vom nicht technisch versierten Anwender ggf. nicht rechtzeitig erkannt bzw. beherrscht werden.

Im Gegensatz zu einer virtuellen Barriere gemäß vorstehender Definition, lässt sich auch ein zu untersuchender Patientenkörper durch eine körperlich existente, rigide Barriere wie beispielsweise einen Käfig von der Vorrichtung zur robotisch geführten Ausrichtung des Magnetfelderzeugers kompakter Bauart separieren, um damit das Risiko von den Patienten gefährdenden Einwirkungen auf den Patientenkörper zu eliminieren (Crash-Schutz). Nachteile dieser technischen Maßnahme sind jedoch, dass die rigide Barriere selbst einen gewissen Raum benötigt, der für die Vorrichtung zur robotisch geführten Ausrichtung des Magnetfelderzeugers kompakter Bauart nicht mehr zur Verfügung steht und diese auch nicht die individuellen anatomischen Gegebenheiten von Patienten berücksichtigen kann, es sei denn, die rigide, körperliche Barriere besitzt Verstellmöglichkeiten, um diese der Patientenanatomie wenigstens anzunähern. Somit ist die für die Annäherung des Magnetfelderzeugers kompakter Bauart vorteilhafte Krafteinwirkung auf den Patientenkörper nicht mehr möglich, d.h. mit dieser technischen Lösung der Anordnung einer körperlichen Barriere ist eine bestmögliche Annäherung des Magnetfelderzeugers kompakter Bauart an das zu steuernde intrakorporale Objekt ebenfalls nicht mehr (in jedem Fall) gegeben.

Insbesondere bei der Anwendung aktuierter (motorisch angetriebener) Systeme in der medizinischen Praxis oder Klinik ist die Berücksichtigung strenger Sicherheitsanforderungen auf Grundlage von Risikobetrachtungen erforderlich. Patientenrisiken durch die robotisch geführte Ausrichtung eines Magnetfelderzeugers kompakter Bauart ergeben sich unter anderem durch die Bewegungen der Vorrichtung zur Positionierung und / oder Ausrichtung des Magnetfelderzeugers kompakter Bauart, die zu Kollisionen mit oder ohne Krafteinwirkung auf den Patientenkörper und damit zu den Patienten gefährdenden Einwirkungen auf den Patientenkörper führen können.

Erschwerend kommt hinzu, dass unter bestimmten Bedingungen vordefinierte Kollisionen mit oder ohne Krafteinwirkung auf den Patientenkörper sogar vorteilhaft sein können, etwa um den Magnetfelderzeuger kompakter Bauart möglichst nahe an das zu steuernde intrakorporale Objekt zu bewegen, wie dies vorstehend bereits angedeutet wurde. In diesem Fall ist eine sensorische Rückkopplung der Krafteinwirkung der Vorrichtung zur robotisch geführten Ausrichtung des Magnetfelderzeugers kompakter Bauart auf den Patientenkörper vorteilhaft/notwendig, um die Krafteinwirkung gezielt zu erzeugen und auch zu begrenzen. Auf diese Weise kann die Krafteinwirkung auf den Patientenkörper in einem Bereich gehalten werden, der nicht gefährdend für den Patienten ist. Das Problem hier ist jedoch, dass Sensoren zur Erfassung der Kollisionssituation ausfallen oder falsche Messergebnisse liefern können, wobei die letztgenannte Fehlfunktion ggf. nicht oder (für den Patienten) zu spät erkannt wird.

Entscheidend für eine Risikobetrachtung einer solchen Vorrichtung ist zudem, welche aktuierte (motorisch erzeugte) Maximalkraft im Falle einer Fehlfunktion auf den Patienten ausgeübt werden kann. Beispielsweise kann eine Fehlfunktion eines Sensors dazu führen, dass die Vorrichtung die Krafteinwirkung auf den Patientenkörper über den, den Patienten nicht gefährdenden Bereich hinaus erhöht, nämlich in einen Bereich, der für den Patienten gefährdend ist. Voraussetzung hierfür ist, dass die in der Vorrichtung verwendeten Aktoren oder motorischen Antriebe wie Elektromotoren, Piezoantriebe, hydraulische bzw. pneumatische Stellkolben, elektromagnetische Antriebe, etc. eine höhere Kraft erzeugen können/müssen, als in der bestimmungsgemäßen Funktion vorgesehen.

Die robotische Führung eines Magnetfelderzeugers kompakter Bauart erfordert die Ausrichtung des Magnetfelderzeugers kompakter Bauart mit (maximal) fünf Freiheitsgraden der Bewegung. Diese fünf Freiheitsgrade sind translatorische Bewegungen entlang der drei rechtwinkelig zueinander stehenden Raumachsen sowie die Rotation um diejenigen Raumachsen, die senkrecht zueinander und vorzugsweise senkrecht zur Polarisationsachse des vom Magnetfelderzeuger kompakter Bauart erzeugten Magnetfeldes stehen und nachstehend als Nick- und Gierbewegung bezeichnet werden. Die Polarisationsachse ist kollinear zur Verbindungslinie zwischen Nord- und Südpol des Magnetfelderzeugers kompakter Bauart. Da eine Rotation des Magnetfelderzeugers kompakter Bauart um diese Polarisationsachse des Magnetfeldes (vorliegend als "Rollbewegung" definiert) zu keiner Änderung des Magnetfeldes im Raum führt, ist diese Ausrichtung des Magnetfelderzeugers kompakter Bauart durch die Vorrichtung zur robotischen Führung desselben nicht zweckdienlich bzw. technisch sinnlos und daher überflüssig.

Vorrichtungen zur robotisch geführten Ausrichtung eines Magnetfelderzeugers kompakter Bauart sind vorzugsweise Roboterarme bzw. Ausleger. Heute bekannte und kommerziell erhältliche Roboterarme sind größtenteils in der Automatisierungstechnik eingesetzt und optimiert auf hohe Verfahrgeschwindigkeiten, hohe Präzision sowie Variabilität in der Bahnführung und Lastenaufnahme.

In der robotisch geführten Ausrichtung eines Magnetfelderzeugers kompakter Bauart zur Steuerung intrakorporaler Objekte sind hingegen die üblichen Verfahrgeschwindigkeiten deutlich geringer als die in der Automatisierungstechnik üblichen Verfahrgeschwindigkeiten. Während in der Automatisierungstechnik Verfahrgeschwindigkeiten in der Größenordnung von bis zu 10 Metern pro Sekunde liegen, werden in der Anwendung einer Vorrichtung zur robotisch geführten Ausrichtung eines Magnetfelderzeugers kompakter Bauart gemäß der vorliegenden Erfindung deutlich geringere Verfahrgeschwindigkeiten benötigt, beispielsweise im Bereich bis 0,1 Meter pro Sekunde. Der Nachteil von heute bekannten und kommerziell erhältlichen Roboterarmen für die Automatisierungstechnik ist, dass die Aktorik auf hohe Geschwindigkeiten ausgelegt ist und daher hohe Kräfte freisetzen können. Außerdem müssen Roboter dieser Gattung häufig schwere Lasten halten und bewegen (einschließlich des Eigengewichts der Roboterarme selbst), sodass hohe Antriebskräfte und/oder Drehmomente erforderlich sind, um die Roboterarme zu bewegen. Aus diesem Grund werden in der Automatisierungstechnik solche Roboterarme ausschließlich innerhalb rigider Barrieren eingesetzt, wie dies vorstehend bereits ausgeführt wurde. In der Anwendung zur robotisch geführten Ausrichtung eines Magnetfelderzeugers kompakter Bauart können Fehlsteuerungen oder Fehlfunktionen daher zu den Patienten gefährdenden Einwirkungen auf den Patientenkörper führen.

In der robotisch geführten Ausrichtung eines Magnetfelderzeugers kompakter Bauart zur Steuerung intrakorporaler Objekte gemäß der vorliegenden Erfindung sind jedoch Anforderungen an die Präzision sowie die Variabilität der Bahnführung und Lastenaufnahme deutlich geringer als in der Automatisierungstechnik heute üblich. Zur akkuraten Ausrichtung eines intrakorporalen Objektes ist eine Präzision der Bewegung des extrakorporalen Magnetfelderzeugers nach kompakter Bauart im Bereich von Millimetern bis einem Zentimeter völlig ausreichend, da sich die Dislokation des Magnetfelderzeugers kompakter Bauart in diesem Bereich eine nur unwesentliche Änderung des Magnetfeldes am Ort des intrakorporalen Objektes ergibt. Des Weiteren sind bei der robotisch geführten Ausrichtung eines Magnetfelderzeugers kompakter Bauart zur Steuerung intrakorporaler Objekte die Anforderungen an Variabilität der Bahnführung aufgrund der auf fünf begrenzten Anzahl der zu aktuierenden Freiheitsgrade sowie des sich um den Patientenkörper erstreckenden limitierten Arbeitsbereichs im Vergleich zu den in der Automatisierungstechnik üblichen Anforderungen eingeschränkt. In der Automatisierungstechnik werden ferner variable Roboter häufig eingesetzt, die sich für unterschiedliche Aufgaben und Manöver eignen und die an unterschiedliche Belastungen angepasst werden können. Dem gegenüber stellt der Magnetfelderzeuger kompakter Bauart gemäß der vorliegenden Erfindung eine konstante, sich nicht verändernde Last dar. Die Vorrichtung zur robotisch geführten Ausrichtung des Magnetfelderzeugers kompakter Bauart hat daher zu jedem Zeitpunkt dieselbe Last, wohingegen in der Automatisierungstechnik unterschiedliche und dynamisch wechselnde Lasten die Regel sind.

In der robotisch geführten Ausrichtung eines Magnetfelderzeugers kompakter Bauart zur Steuerung intrakorporale Objekte sind die Anforderungen an Präzision, Variabilität der Bahnsteuerung und Lastenaufnahme, wie oben beschrieben, im Vergleich zu den Anforderungen in der Automatisierungstechnik deutlich geringer. Der Einsatz eines Roboters aus der Automatisierungstechnik als Vorrichtung zur robotisch geführten Ausrichtung eines Magnetfelderzeugers kompakter Bauart zur Steuerung intrakorporaler Objekte hat daher den grundsätzlichen Nachteil, dass die Auslegung des Roboterarms die Anforderungen des Einsatzes als Vorrichtung zur robotisch geführten Ausrichtung eines Magnetfelderzeugers kompakter Bauart gemäß vorliegender Erfindung zur Steuerung intrakorporaler Objekte übererfüllen, derart, dass diese eine Gefahr für den Patienten und das Bedienpersonal darstellen. Die Leistungsdaten der Aktorik eines solchen Roboters, der für die Automatisierungstechnik konzipiert wurde, ermöglichen zudem den Patienten gefährdende Einwirkungen auf den Patientenkörper.

Grundsätzlich besteht in der Fachwelt die Bestrebung, durch so genanntes "Down-Sizing" überdimensionierte Vorrichtungen so zu verkleinern oder in ihrer Leistung zu reduzieren, dass diese gerade noch die Aufgaben erfüllen können, die ihnen zugedacht sind. Im vorliegenden Fall aber sind die Anforderungen an die Robotik bzw. die magnetische Führungseinrichtung gegensätzlich, dahingehend, dass diese zum Einen keine Gefahr für den Patienten und/oder das Bedienpersonal darstellen dürfen und zum Anderen jedoch robust genug sein müssen, um einem permanenten manuellen (auch unsachgemäßen) Umgang mit allen Widrigkeiten in einer Praxis oder Klinik Stand zu halten. Ein einfaches "Down-Sizing" nach bekannten Vorbildern würde daher nicht zu einem befriedigenden Ergebnis führen.

### Aufgabe der Erfindung

Angesichts dieser Problematik besteht die Aufgabe der vorliegenden Erfindung darin, eine Vorrichtung zur robotisch geführten Ausrichtung eines Magnetfelderzeugers kompakter Bauart insbesondere eine extrakorporale Führungseinrichtung eines intrakorporalen magnetischen Objekts wie beispielsweise einer Endoskopkapsel zu schaffen, die im Hinblick auf die Patientensicherheit optimiert ist gleichzeitig aber die Durchführung der erforderlichen Manöver zulässt, ohne die Einschränkungen, die von den oben erläuterten technischen Lösungen bekannt sind.

### Kurzbeschreibung der Erfindung

Diese Aufgabe wird prinzipiell durch eine (magnetische) extrakorporale Führungseinrichtung (vorzugsweise in Form einer Knickarm-/Scara-Robotik ggf. kombiniert mit ausziehbaren Roboterarmen) mit den Merkmalen des Patentanspruchs 1 gelöst. Vorteilhafte Ausgestaltungen der Erfindung sind Gegenstand der Unteransprüche.

Der Kern der Erfindung besteht demzufolge darin, durch bestimmte technische Maßnahmen die Einrichtung zur robotisch geführten Ausrichtung eines Magnetfelderzeugers kompakter Bauart so zu gestalten, dass wahlweise die Ausrichtung der einzelnen Freiheitsgrade des Magnetfelderzeugers kompakter Bauart insgesamt oder in ausgewählter Weise vom Patientenkörper separiert, d.h. abgeschirmt werden und/oder die Krafteinwirkung auf den Patientenkörper bei Betätigung der einzelnen Freiheitsgrade vorzugsweise über die Auslegung der jeweiligen Antriebe zur Betätigung der einzelnen Freiheitsgrade begrenzt werden. Durch die Umsetzung dieser prinzipiellen technischen Maßnahmen lässt sich die Vorrichtung im Fall einer medizinischen Applikation in direktem Kontakt mit dem Patientenkörper verwenden, wobei für den Patienten schädliche Einwirkungen auf den Patientenkörper weitgehend ausgeschlossen werden können

In anderen Worten ausgedrückt, liegt der Erfindung die Überlegung zugrunde, jene Freiheitsgrade einer Robotik oder Führungseinrichtung zu identifizieren, die in integraler Weise (d.h. an der Führungseinrichtung) eingehaust bzw. eingesperrt werden können ohne die Funktion der Einrichtung zu beschränken, d.h. die robotischen Bewegungen bezüglich der identifizierten Freiheitsgrade finden in einem individuellen, Führungseinrichtungs-internen Gehäuse oder innerhalb einer internen Absperrung statt, um so die Anzahl an von Außen frei zugänglichen Freiheitsgraden zu verringern und damit ein Verletzungsrisiko durch die Robotik insgesamt senken. Je nach Auswahl der für die interne Einhausung/Einsperrung vorgesehenen Freiheitsgrade lässt sich ein Kollisionsrisiko der verbleibenden freien Freiheitsgrade weiter reduzieren. D.h. bevorzugt werden jene Freiheitsgrade (intern) eingehaust/eingesperrt, welche besondere Risiken für eine Bedienperson, oder im Fall einer medizinischen Applikation, für einen Patienten bergen.

Darüber hinaus können zumindest ausgewählte Freiheitsgrade zusätzlich oder alternativ im Wesentlichen kraftfrei gestaltet werden. D.h., das Risiko einer Kollision/Verletzung, die von einem Freiheitsgrad einer Robotik ausgeht, ist nur so hoch wie die Kraft, die zur Aktivierung/Aktuierung des Freiheitsgrads erforderlich ist. Diese Kraft ist abhängig von der Belastung, die auf einen Aktor (motorischen Antrieb) des Freiheitsgrads einwirkt. Als eine Konsequenz aus dieser Überlegung ergibt sich, die Betriebsbelastung so klein wie möglich zu halten, indem unnötige Lasten, die auf den Antrieb einwirken, wie beispielsweise interne Lasten der Robotik selbst, außerhalb bzw. unter Umgehung des Antriebs abgefangen werden. In diesem Fall kann der Antrieb im Wesentlichen nur zur Überwindung von Trägheitskräften ausgelegt werden.

Konkreter ausgedrückt wird die gestellte Aufgabe durch eine Robotik, vorzugsweise eine (magnetische) Führungseinrichtung für ein intrakorporales Objekt gelöst, mit einer motorisch angetriebenen Positioniervorrichtung (Roboterarmsystem vorzugsweise nach dem Knickarm- und/oder Scaraprinzip), die maximal drei zu aktivierende Freiheitsgrade zur vorzugsweise translatorischen Bewegung eines Anschlussinterface der Positioniervorrichtung hat, an das ein (magnetischer) Endeffektor angeschlossen oder anschließbar ist, der maximal zwei zu aktivierende Freiheitsgrade zur vorzugsweise rotatorischen Bewegung eines Magnetfelderzeugers hat. Der Begriff "Freiheitsgrad" betrifft vorliegend ein Gelenk/Scharnier/Auszug etc. vorzugsweise zur Verwirklichung einer Bewegung in einer einzigen Ebene. Erfindungsgemäß ist zumindest einer der zwei Freiheitsgrade des Magnetfelderzeugers (oder beide) in einem (einzigen) Effektorgehäuse und/oder Gitterrahmen eingehaust bzw. ein-/abgesperrt.

Das bedeutet, dass für den Fall, dass nur ein Freiheitsgrad eingehaust/eingesperrt ist, der Magnetfelderzeuger innerhalb dieses Gehäuses/Rahmens eine Bewegung in einer Ebene (gemäß einem Freiheitsgrad) ausführt, die nicht nach außen dringt bzw. nach außen abgeschirmt ist. Der zweite (freiliegende) Freiheitsgrad bedeutet dann zwangsläufig eine (in einer Ebene stattfindende) Bewegung des Gehäuses/Rahmens selbst und mit diesem zusammen eine gleiche/gemeinsame (Mit-) Bewegung des Magnetfelderzeugers. Vorteilhaft ist es aber, die zwei (rotatorischen) Freiheitsgrade des Magnetfelderzeugers in einem einzigen Gehäuse/Rahmen einzuhausen/einzusperren und damit nach Außen zu isolieren/abzuschirmen. In diesem Fall würde das (einzige) Gehäuse/Rahmen des Endeffektors ausschließlich durch die Robotik der Positioniervorrichtung (dreidimensional) translatorisch bewegt. Ein Verschwenken des Gehäuses um eine seiner Achse, ausgelöst durch die Positioniervorrichtung, ist nicht vorgesehen. Durch diese Ausführung lassen sich Risiken einer unbeabsichtigten Kollision mit einer nahen Bedienperson während des Betriebs der Robotik bzw. der Führungseinrichtung reduzieren, da die Anzahl der nach Außen exponierten Freiheitsgrade/Bewegungen verringert ist.

Ein zusätzlicher oder unabhängiger Aspekt der Erfindung sieht vor, dass die drei Freiheitsgrade der Positioniervorrichtung (ausschließlich) eine translatorische Bewegung des Anschlussinterface in eine X-, Y- und Z-Richtung eines Raum-Koordinatensystems erlauben (Freiheitsgrade a, b und c), in welchem die Y-Achse vorzugsweise im Wesentlichen längs der Schwerkraftrichtung ausgerichtet ist. Zusätzlich werden Magnetfelderzeuger-interne Achsen A und B festgelegt, die zusammen mit der Polarisationsachse (C-/Längsachse) des Magnetfelderzeugers die Ausrichtung bzw. Orientierung des Magnetfelderzeugers relativ zum Raum-Koordinatensystem (X-, Y- und Z-Achse) beschreiben.

Damit wird jeweils eine Rotationsbewegung des Magnetfelderzeugers um eine Achse A und B des Relativ-Koordinatensystems (A-, B- und C-Achse) erlaubt, wobei die Achse B vorzugsweise im Wesentlichen horizontal und senkrecht zur Polarisationsachse (C-Achse) des Magnetfelderzeugers ausgerichtet ist, so dass eine Rotationsbewegung des Magnetfelderzeugers um die Achse B ein "Nicken" der Polarisationsachse bewirkt (Freiheitsgrad e), und wobei unabhängig von der aktuellen Ausrichtung der Achse A (infolge eines Nickens) eine Rotationsbewegung des Magnetfelderzeugers um die Achse Y des Raum-Koordinatensystems ein "Schwenken" (bzw. Gieren) des Magnetfelderzeugers (und damit des Magnetfeldes) bewirkt (Freiheitsgrad d).

Steht im Fall einer extremen Nickbewegung die Polarisationsachse kollinear zur Achse Y, bewirkt eine Rotationsbewegung des Magnetfelderzeugers um die Achse Y keine wesentliche Änderung des Magnetfeldes mehr.

Um den vorstehenden technischen Zusammenhang nochmals in anderer Form auszudrücken sei nachfolgen eine 0-Lage (entspricht beispielsweise einer horizontalen Ausrichtung) des Magnetfelderzeugers betrachtet:
Wie vorstehend ausgeführt ist, erlauben die drei Freiheitsgrade der Positioniervorrichtung (ausschließlich) eine translatorische Bewegung des Anschlussinterface in eine X-, Y- und Z-Richtung eines Raum-Koordinatensystems, in welchem die Y-Achse vorzugsweise im Wesentlichen längs der Schwerkraftrichtung und die Z-Achse demzufolge entlang der Längs- und damit der Polarisationsachse des Magnetfelderzeugers im Fall seiner horizontalen Raumlage (0-Lage) ausgerichtet ist, wohingegen die (ausschließlich) zwei Freiheitsgrade des Magnetfelderzeugers jeweils eine Rotationsbewegung des Magnetfelderzeugers um jeweils eine Achse vorzugsweise um die Y- und X-Achse erlauben, um ein Gieren und Nicken des Magnetfelderzeugers bezüglich des Raum-Koordinatensystems zu bewirken.

Das heißt, es wird ein Raum-Koordinatensystem für die Führungseinrichtung festgelegt, in welchem die Z-Achse der Polarisations- und damit der Längsachse des Magnetfelderzeugers in dessen 0-Lage entspricht, wohingegen die Y-Achse die Hochachse (vorzugsweise längs der Schwerkraftrichtung) und die X-Achse die Querachse des Magnetfelderzeugers darstellen. Ausgehend von dieser 0-Lage kann dann der Magnetfelderzeuger um die Y- und X-Achse gedreht werden, wohingegen eine Drehung des Magnetfelderzeugers um die Z-Achse (Polarisationsachse) ggf. theoretisch möglich, aber weil hierdurch keine Änderungen des Magnetfelds eintreten (d.h. technisch sinnlos) nicht vorgesehen ist.

Diese Maßnahme der Verlagerung der Rotations-Freiheitsgrade ausschließlich auf den Endeffektor gemäß vorstehender Beschreibung vereinfacht das Bewegungsmuster der Positioniereinrichtung auf rein translatorische Bewegungen des Anschlussinterface, die von einer Bedienperson erwartet bzw. einfacher vorhergesehen werden können. Die diese überlagernden rotatorischen Bewegungen sind von der Positioniervorrichtung getrennt und zumindest teilweise eingehaust/eingesperrt. Damit bleibt die nach außen exponierte Motorik (Bewegungsdynamik) der Führungseinrichtung insgesamt überschaubar.

Eine alternative vorteilhafte Ausgestaltung der Erfindung sieht vor, dass die zwei Freiheitsgrade des Magnetfelderzeugers jeweils eine Rotationsbewegung des Magnetfelderzeugers um die eine Achse A und die eine Achse B erlauben, wobei die Achse B im Wesentlichen horizontal und senkrecht zur Polarisationsachse des Magnetfelderzeugers ausgerichtet ist, so dass eine Rotationsbewegung des Magnetfelderzeugers um die Achse B ein "Nicken" der Polarisationsachse bewirkt (Freiheitsgrad e), und wobei die Achse A im Wesentlichen senkrecht sowohl zur Achse B also auch der Polarisationsachse des Magnetfelderzeugers steht, so dass eine Rotationsbewegung des Magnetfelderzeugers um die Achse A ein "Gieren" der Polarisationsachse des Magnetfelderzeugers bewirkt (Freiheitsgrad d). Eine Rotationsbewegung des Magnetfelderzeugers um die Polarisationsachse ist auch hier ggf. theoretisch möglich, aber nicht vorgesehen, weil hierdurch keine wesentliche Änderungen des Magnetfelds eintritt (d.h. technisch sinnlos). Die Vorteile dieser Variante sind die Gleichen, wie zuvor beschieben, sodass an dieser Stelle auf die vorstehenden Textstellen verwiesen werden kann.

Ein anderer oder zusätzlicher Aspekt der Erfindung betrifft die besondere Auslegung des Antriebs, dahingehend, dass die Positioniervorrichtung eine Anzahl von jeweils motorisch angetriebenen Auslegern oder Armen hat, von denen zumindest ausgewählte Ausleger oder Arme bezüglich der jeweils zugehörigen motorischen Antriebe insbesondere im Fall einer Knickarmrobotik gewichtsausbalanciert sind. Vorzugsweise ist hierfür ein solches Balanciersystem vorgesehen, das eine dynamische oder zumindest gestufte Anpassung (Annäherung) der Gewichtsausbalancierung an die Gewichtsbelastung vornimmt oder bewirkt, welche aktuell auf den jeweiligen motorischen Antrieb einwirkt.

Beispielsweise ist im Fall eines schwenkbaren Auslegers/Arm die statische Belastung des Antriebs abhängig vom aktuellen Anstellwinkel des Auslegers bezüglich der Schwerkraftrichtung. D.h. bei horizontaler Erstreckung wird ein Antrieb maximal gewichtsbelastet. Es liegt auf der Hand, dass die Antriebskraft so groß sein muss, um eine Bewegung des Auslegers und daran befindlicher Lasten aus jeder Winkelposition zu gewährleisten. Dieser Antrieb wäre somit bezüglich einer Winkelposition unterschiedlich zur horizontalen Ausrichtung überdimensioniert und daher prinzipiell in der Lage, eine Person zu verletzen. Sobald jedoch der Ausleger quasi gewichtslos (ausbalanciert) ist, braucht der Antrieb nur noch die Massenträgheit zu überwinden. Er kann somit deutlich kleiner/schwächer ausgelegt sein.

Vorteilhaft ist es demnach, wenn die Positioniervorrichtung eine Anzahl von jeweils motorisch angetriebenen Auslegern oder Armen hat, von denen zumindest ausgewählte Ausleger oder Arme bezüglich der zugehörigen motorischen Antriebe so gelagert sind, dass die motorischen Antriebe keine statische Gewichtslast aufnehmen oder überwinden müssen. Dies kann prinzipiell erreicht werden durch den Einsatz eines Gegenkraftsystems (z.B. das Anbringen von Gegengewichten und/oder Federn an einer Hebelarm-Kinematik, etc.) oder indem die vorbestimmte Bewegungsrichtung der Ausleger vorzugsweise im Wesentlichen senkrecht zur Schwerkraft weist (gemäß dem Prinzip einer Scara-Robotik). Im letzteren Fall würde die statische Gewichtskraft von einem entsprechend ausgebildeten Loslager parallel zum Antrieb aufgenommen werden, wodurch der Antrieb selbst entlastet wäre.

Gemäß einem anderen oder zusätzlichen Aspekt der Erfindung ist der jeweilige motorische Antrieb für ausgewählte Freiheitsgrade antriebskraftbegrenzt (z.B. durch Anordnung einer Rutschkupplung, einer Sicherheitskupplung, eines Schrittmotors, der bei einer bestimmten Kraft außer Tritt fällt, eines Überdruckventils, etc.) derart, dass die maximal abgebbare Antriebskraft gleich oder größer ist als eine zu erwartende Betriebs-Belastungskraft, jedoch gleich oder kleiner ist als eine vorbestimmte Maximalkraft, bei der eine Verletzung eines Patienten oder einer Bedienperson im Kollisionsfall weitgehend ausgeschlossen wird. Eine solche Maximalkraft ist bereits im Stand der Technik beispielsweise gemäß EN ISO 10218 definiert, sodass an dieser Stelle auf die entsprechenden Normen verwiesen werden kann.

Unabhängig von den vorstehend beschriebenen Sicherungssystemen/Maßnahmen wäre es vorteilhaft, wenn eine Überwachungseinrichtung vorgesehen ist, an die eine Anzahl von Sensoren zur Überwachung des Betriebszustands der Führungseinrichtung und ggf. zur Abschaltung der Führungseinrichtung bei erfasstem vorbestimmten Unfallrisiko angeschlossen oder anschließbar ist. Vorzugsweise sind die Sensoren ausgewählt/auswählbar aus einer Gruppe von Sensoren umfassend:
- ein Kraftsensor zur Erfassung der Antriebslast auf den jeweiligen (ausgewählten) Antrieb,
- ein Berührungssensor zur Erfassung der Kontaktkraft zwischen dem magnetischen Endeffektor oder einem ausgewählten Ausleger der Positioniervorrichtung und einer Bedienperson oder einem Patienten,
- ein Deformations-Erfassungssensor zur Erfassung beispielsweise einer Eindellung vorzugsweise am Endeffektorgehäuse,
- ein optischer Sensor zur optischen Erfassung von Hindernissen und
- ein Abstandssensor zur Erfassung eines Abstands zu einem Hindernis

Alternativ hierzu oder zusätzlich kann die magnetische Führungseinrichtung auch mit Bewegungsbegrenzungsmittel vorzugsweise in Form von programmierbaren und/oder mechanischen Endanschlägen/Endschaltern ausgerüstet sein, welche so an der Positioniervorrichtung und/oder dem magnetischen Endeffektor montiert sind, dass zumindest eine Auswahl der maximal drei Freiheitsgrade der Positioniervorrichtung und/oder der maximal zwei Freiheitsgrade des Magnetfelderzeugers auf einen vorbestimmten Bewegungsbereich mechanisch eingeschränkt ist.

Wie vorstehend bereits angedeutet ist, soll das Gehäuse des magnetischen Endeffektors in vorteilhafter Weise hinsichtlich seiner Ausrichtung unverändert bleiben. Vorzugsweise weist in diesem Fall das Gehäuse des magnetischen Endeffektors unabhängig von der aktuellen Bewegung der Positioniervorrichtung und/oder des Magnetfelderzeugers immer im Wesentlichen in Schwerkraftrichtung. In anderen Worten ausgedrückt, ist der Magnetfelderzeuger innerhalb der Gehäuses/Rahmens des Endeffektors so gelagert, dass dessen Gierachse in 0-Lage (senkrecht zur nunmehr horizontalen Polarisationsachse) in Schwerkraftrichtung weist. Dies hat den Vorteil, dass eine Bedienperson keine auskragende Bewegung / Verschwenkung des Gehäuses selbst beachten muss, wodurch das Verletzungsrisiko weiter sinkt.

### Kurzbeschreibung der Ausführungsbeispiele

Die Erfindung wird nachstehend anhand bevorzugter Ausführungsbeispiele unter Bezugnahme auf die begleitenden Figuren näher erläutert.
Fig. 1 zeigt das konstruktive Grundprinzip einer (magnetischen) Führungseinrichtung oder eines (medizinischen) Roboterarms gemäß einem ersten bevorzugten Ausführungsbeispiel der Erfindung mit der Einhausung zweier ausgewählter Freiheitsgrade (ein Freiheitsgrad entspricht der erfindungsgemäßen Definition eines Gelenks oder Scharniers zur Erreichung einer eindimensionalen Bewegung bzw. Rotation um eine Achse),
Fig. 2 zeigt das konstruktive Grundprinzip einer (magnetischen) Führungseinrichtung oder eines (medizinischen) Roboterarms gemäß einem zweiten bevorzugten Ausführungsbeispiel der Erfindung als Alternative zum Ausführungsbeispiel gemäß der Fig. 1 mit der Einhausung eines einzigen ausgewählten Freiheitsgrades,
Fig. 3 zeigt das konstruktive Grundprinzip einer (magnetischen) Führungseinrichtung oder eines (medizinischen) Roboterarms gemäß einem dritten bevorzugten Ausführungsbeispiel der Erfindung mit der Einhausung eines (einzigen) ausgewählten Freiheitsgrades sowie mit einer konstruktiven Roboterarmanordnung mit möglichst geringer Belastung der Aktorik durch Eigengewicht,
Fig. 4 zeigt das konstruktive Grundprinzip einer (magnetischen) Führungseinrichtung oder eines (medizinischen) Roboterarms gemäß einem vierten bevorzugten Ausführungsbeispiel der Erfindung mit der Einhausung zweier ausgewählter Freiheitsgrade sowie mit einer konstruktiven Roboterarmanordnung mit möglichst geringer Belastung der Aktorik durch Eigengewicht und zusätzlich oder alternativ einer gewichtsausgeglichenen (im Wesentlichen dynamisch ausbalancierten) Roboterarmanordnung,
Fig. 5 zeigt das konstruktive Grundprinzip einer (magnetischen) Führungseinrichtung oder eines (medizinischen) Roboterarms gemäß einem fünften bevorzugten Ausführungsbeispiel der Erfindung mit einer im Wesentlichen dynamisch ausbalancierten Roboterarmanordnung zur Verwendung beispielsweise in einer der Ausführungsbeispiele gemäß der Fig. 1 und 2,
Fig. 6 zeigt eine Kräftetabelle zum Vergleich einer Roboterarmkonstruktion mit im Wesentlichen vertikalen Drehachsen der einzelnen Arme, einer Roboterarmkonstruktion mit im Wesentlichen horizontalen Drehachsen der einzelnen Arme und einer Roboterarmkonstruktion mit im Wesentlichen horizontalen Drehachsen sowie einer dynamischen Balancierung (Gewichtskompensation),
Fig. 7a-7b zeigen jeweils die Prinzipdarstellung eines Magnetfelderzeugers (Permanentmagneten) zur Definition der fünf Freiheitsgrade, in die er mittels der erfindungsgemäßen Positioniervorrichtung (der Fig. 1 bis 5) und des daran angeschlossenen Endeffektors maximal bewegbar sein soll,
Fig. 8 zeigt den magnetischen Endeffektor einschließlich des darin gelagerten Magnetfelderzeugers mit seinen grundsätzlichen Funktionen,
Fig. 9 zeigt den magnetischen Endeffektor mit einer grundsätzlichen konstruktiven Ausgestaltung eines Magnetfelderzeugers des Endeffektors,
Fig. 10a-10e zeigen eine Mehrzahl alternativer Formen für einen Magnetfelderzeuger gemäß der Erfindung, wie er in dem Endeffektor verbaut werden kann,
Fig. 11a-11c zeigen jeweils einen magnetischen Endeffektor mit Gehäuse sowie daran unterschiedlich angebrachter Sensorik,
Fig. 12 zeigt einen weiteren magnetischen Endeffektor mit einer Sensorik zwischen Antrieb (Aktuator) und Magnetfelderzeuger,
Fig. 13 zeigt einen weiteren magnetischen Endeffektor mit Gehäuse sowie einem daran angeordnetem Bedienfeld,
Fig. 14 zeigt die Prinzipdarstellung einer Positioniervorrichtung gemäß der Erfindung zur Aktivierung ausschließlich translatorischer Freiheitsgrade (gemäß der Fig. 1-5) mit einem Anschlussinterface für einen magnetischen Endeffektor gemäß einem der Fig. 7 bis 13,
Fig. 15a -15c zeigen die Positioniervorrichtung bzw. einen Bewegungsteil der Positionsvorrichtung bestehend aus individuell eingehaustem Arm und Aktuator mit daran (am Gehäuse) unterschiedlich angebrachten Sensoren,
Fig. 16 zeigt die Positioniervorrichtung bzw. einen Bewegungsteil der Positionsvorrichtung bestehend aus individuell eingehaustem Arm und Aktuator mit daran (am Gehäuse) angebrachtem Bedienfeld,
Fig. 17a - 17c zeigen unterschiedliche prinzipielle Ausgestaltungen für eine Aktuator - Verbindungselement - Gelenk - Einheit gemäß der Erfindung,
Fig. 18a - 18c zeigen alternative prinzipielle Ausgestaltungen für eine Aktuator - Verbindungselement - Gelenk - Einheit gemäß der Erfindung und
Fig. 19a bis 19f zeigen alternative Ausgestaltungen für Aktuatoren gemäß der Erfindung zur Aktuierung von Freiheitsgraden der Positioniervorrichtung und/oder des magnetischen Endeffektors.

### Detaillierte Beschreibung der Ausführungsbeispiele

### Positionsvorrichtung

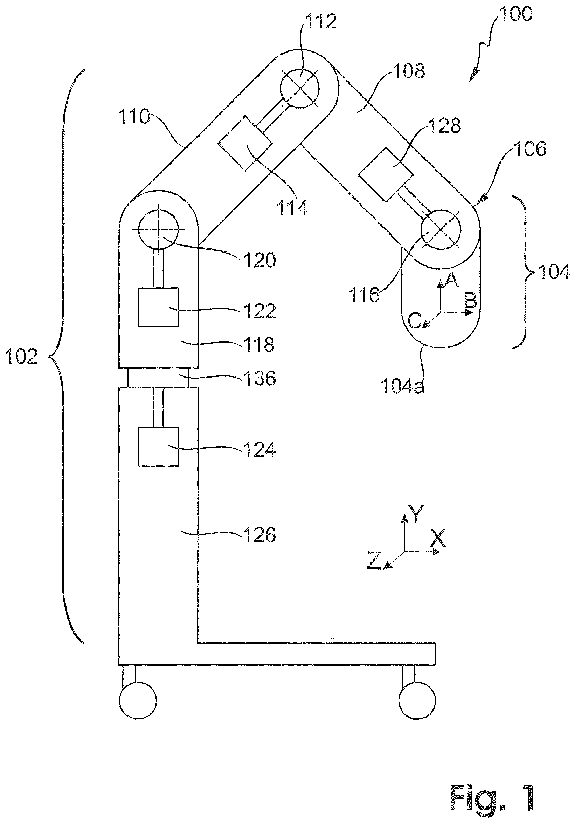

In der Fig. 1 ist ein erstes Ausführungsbeispiel einer magnetischen Führungseinrichtung 100 gemäß der Erfindung prinzipiell dargestellt. Demzufolge besteht die Führungseinrichtung 100 zunächst aus einer Positioniervorrichtung (Roboterarmsystem) 102 und einem magnetischen Endeffektor 104, der an einem Anschlussinterface 106 der Positioniervorrichtung 102 angeschlossen ist.

Die Positioniervorrichtung 102 des ersten Ausführungsbeispiels der Erfindung hat zwei seriell aneinander anscharnierte Schwenkarme 108, 110, deren (im Wesentlichen horizontal ausgerichtetes) verbindendes Scharniergelenk 112 motorisch (per Aktuator 114) aktivierbar ist. Am freien Ende des einen (freien) Schwenkarms 108 ist das Anschlussinterface 106 mittels eines weiteren horizontalen Scharniers 116 angelenkt, wohingegen das freie Ende des anderen (eingebundenen) Schwenkarms 110 an einem Rotationsarm 118 schwenkbar ebenfalls mittels eines horizontalen Scharniers 120 anscharniert ist. Auch dieses Scharniergelenk 120 zwischen dem anderen (eingebundenen) Schwenkarm 110 und dem Rotationsarm 118 ist motorisch (per Aktuator 122) aktivierbar. Der Rotationsarm 118 ist schließlich um seine Längsachse (vertikal) motorisch (per Aktuator 124) drehbar an einer Basis 126 gelagert, die vorliegend auf Rollen abgestützt ist, um vorzugsweise manuell verfahren werden zu können.

Durch diesen Arm/Ausleger-Gelenkmechanismus ist die Positioniervorrichtung 102 in der Lage, das Anschlussinterface 106 translatorisch dreidimensional zu bewegen. D.h. die Positioniervorrichtung 102 gemäß dem ersten bevorzugten Ausführungsbeispiel der Erfindung hat ausschließlich drei sinnvoll zu aktivierende Freiheitsgrade, nämlich für eine translatorische Bewegung des Anschlussinterface 106 in die X-, Y- und Z-Richtung eines (beliebig definierbaren, vorzugsweise jedoch mit Y-Achse längs der Schwerkraftrichtung) Raumkoordinatensystems.

Im vorliegenden Fall ist der magnetischen Endeffektor 104 an dem Anschlussinterface 106 mechanisch sowie elektrisch (pneumatisch und/oder hydraulisch) angedockt, das über das motorisch (per Aktuator 128) aktivierbare horizontale Scharnier 116 an dem einen (freien) Schwenkarm 108 gelagert ist. Wie nachfolgend noch im Einzelnen ausgeführt ist, wird dieses Scharnier 116 vorzugsweise derart aktiviert, dass der magnetische Endeffektor 104 immer in eine bestimmte Richtung bezüglich der Vertikalen weist, weiter vorzugsweise (immer) in Vertikalrichtung ausgerichtet wird. Alternativ hierzu ist es aber auch möglich, dass das horizontale Scharnier 116 zwischen Anschlussinterface 106 und magnetischen Endeffektor 104 keinen motorischen Antrieb hat, sodass sich der magnetische Endeffektor 104 ausschließlich infolge der auf ihn einwirkenden Schwerkraft unabhängig von der aktuellen Ausrichtung der Positionsvorrichtung 102 immer senkrecht ausrichtet.

Im Fall des Ausführungsbeispiels gemäß der Fig. 1 hat der magnetische Endeffektor 104 ein einziges Effektorgehäuse 104a, das fest mit dem Anschlussinterface 106 mechanisch und elektrisch (pneumatisch und/oder hydraulisch) verbunden ist und somit (bezüglich des Anschlussinterface 106) senkrecht nach unten weist (d.h. unterhalb des Anschlussinterface 106 angeordnet ist). Innerhalb des einzigen Effektorgehäuses 104a ist ein in der Fig. 1 nicht weiter gezeigter, nachfolgend jedoch noch detaillierter beschriebener Magnetfelderzeuger 1 untergebracht, dessen Lagerung im vorliegenden Fall genau zwei sinnvoll zu aktivierende Freiheitsgrade hat, nämlich für ein "Nicken" und "Schwenken/Gieren" des Magnetfelderzeugers 1 bezüglich eines Raum-Koordinatensystems.

Die Begriffe "Nicken" und "Gieren" kommen grundsätzlich aus der Luftfahrttechnik und beschreiben dort die Bewegung eines Flugobjekts bezogen auf das Flugobjekt-interne Koordinatensystem. In der vorliegenden Beschreibung sollen jedoch die Bewegungen "Nicken" und "Gieren" auf das Raum-Koordinatensystem bezogen sein.

Gemäß dem Ausführungsbeispiel der Fig. 1 sind die beiden Freiheitsgrade (bzw. Scharniere/Gelenke) für ein "Nicken" (Freiheitsgrad e) und "Schwenken" bzw. "Gieren" (Freiheitsgrad d) des Magnetfelderzeugers 1 nach vorstehender Definition in dem Effektorgehäuse 104a eingehaust/untergebracht und damit von Außen nicht mehr zugänglich. In diesem Fall bewegt sich das Effektorgehäuse 104a quasi einstückig mit dem Anschlussinterface 106 im wesentlichen rein translatorisch in die vorstehend bezeichneten X-, Y-, Z-Richtungen der Positioniervorrichtung 102, wobei die Rotationsbewegungen des Magnetfelderzeugers 1 für dessen Nick- und Schwenk- bzw. Gierbewegung innerhalb des vorzugsweise immer senkrecht ausgerichteten Effektorgehäuses 104a stattfinden.

An dieser Stelle sei der Begriff "senkrecht ausgerichtetes Effektorgehäuse" wie Folgt definiert:
Wie in der Fig. 1 dargestellt ist, hat das Gehäuse an einem (in Schwerkraftrichtung) unteren Endabschnitt eine abgerundete Außenkontur. Diese stellt eine so genannte Kontaktseite/Fläche für ein in Kontakt kommen mit einem Patientenkörper dar. Diese Kontaktseite befindet sich vorzugsweise immer an der (in Schwerkraftrichtung gesehenen) Unterseite des Gehäuses und definiert so die Vertikalausrichtung des Gehäuses. Dabei sei aber auch darauf hingewiesen, dass die Kontaktseite auch die Vertikalseite (gemäß der vorstehenden Definition) sein kann, beispielsweise dann, wenn ein Patient eine stehende oder sitzende Position einnimmt anstelle einer liegenden Position.

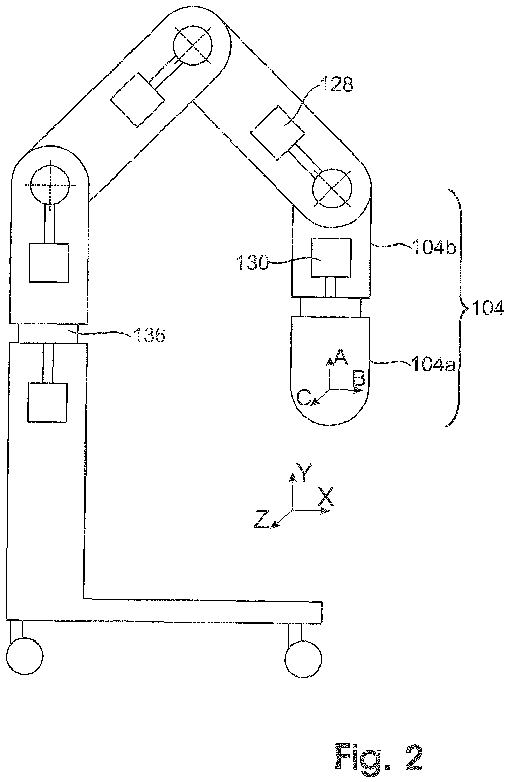

Eine Alternative zu dem Ausführungsbeispiel gemäß der Fig. 1 ist in der Fig. 2 dargestellt, wonach die Positioniervorrichtung 102 einen konstruktiven Aufbau aufweist, wie er bereits vorstehend anhand der Fig. 1 beschrieben wurde. Unterschiedlich hierzu ist jedoch die Ausgestaltung des magnetischen Endeffektors 104, der nunmehr zwei Gehäuse 104a, 104b hat, von denen das eine Gehäuse 104b fest mit dem Anschlussinterface 106 der Positioniervorrichtung 102 mechanisch/elektrisch/hydraulisch/pneumatisch verbunden ist und infolge der Schwerkrafteinwirkung oder aktiv durch einen entsprechend angesteuerten Autor 128 (gemäß der Fig. 2) immer senkrecht (im Sinne der vorstehenden Definition) nach unten zeigt. An dem einen, am Anschlussinterface 106 montierten Gehäuse 104b ist ein weiteres Gehäuse 104a motorisch (per Aktuator 130) drehbar um die Gehäuselängsachse (in diesem Fall die Vertikalachse) gelagert. In dem weiteren Gehäuse 104a ist der Magnetfelderzeuger untergebracht, derart, dass er eine motorisch angetriebene Nickbewegung bezüglich der Gehäuselängsachse ausführen kann. In dem vorliegenden Fall findet die Gierbewegung des Magnetfelderzeugers demnach durch eine motorisch angetriebene Rotation des weiteren Gehäuses 104a bei rotatorisch ruhendem einen Gehäuse 104b statt. D.h. im Fall der Fig. 2 ist lediglich der eine Freiheitsgrad (Scharnier) für eine Nickbewegung des Magnetfelderzeugers im Effektorgehäuse eingehaust/untergebracht, wohingegen der Freiheitsgrad (Scharnier/Drehachse) für eine Gierbewegung des Magnetfelderzeugs, ausgeführt durch Rotieren des weiteren Effektorgehäuses 104b von Außen frei zugänglich ist.

An dieser Stelle sei nochmals die Bewegungen des Magnetfelderzeugers 1 gemäß der ausschließlich zwei technisch sinnvollen Freiheitsgrade anhand der Fig. 1 und 2 beispielhaft erläutert.

Gemäß dem bevorzugten Ausführungsbeispiel der Erfindung ist es vorgesehen, dass das Gehäuse 104a,b so ausgerichtet ist, dass dessen Kontaktseite mit dem (nicht weiter dargestellten) Patienten senkrecht nach unten weist. Demzufolge orientiert sich das äußere Raum-Koordinatensystem mit der X-, Y- und Z-Achse nach der Schwerkraftrichtung. D.h. die Y-Achse ist linear zur Schwerkraftrichtung.

Obgleich eine Ausgangs- oder 0-Lage des Magnetfelderzeugers 1 (z.B. ein Permanentmagnet) innerhalb des Gehäuses 104a,b unabhängig vom Raum-Koordinatensystem und damit der Ausrichtung des Gehäuses 104a,b sein kann, wird beispielhaft angenommen, dass eine interne Polarisationsachse C (Längsachse) des Magnetfelderzeugers 1in 0-Lage in Z-Richtung des eingezeichneten Raum-Koordinatensystems erstreckt (also horizontal verläuft). Eine hierzu senkrechte interne Achse A des Magnetfelderzeugers 1 verläuft dann in 0-Lage in Schwerkraftrichtung und eine hierzu senkrechte interne Achse B des Magnetfelderzeugers 1 verläuft dann in 0-Lage ebenfalls horizontal längs der X-Achse des Raum-Koordinatensystems. Wenn der Magnetfelderzeuger 1 nickt, dann dreht dieser um die interne Achse B, d.h. um die Raum-Koordinatenachse X. Wenn der Magnetfelderzeuger 1 zusätzlich giert, dann schwenkt er in jedem Fall um die Raum-Koordinatenachse Y aber nur noch anteilsmäßig um die bereits genickte interne Achse A. Dieser Anteil wird zwangsläufig kleiner je größer der Nickwinkel ist und letztlich 0, wenn die interne Achse C (Polarisationsachse) in Schwerkraftrichtung weist. Dann wird nämlich die Rotation um die Y-Achse zu einer Rollbewegung ohne Einfluss auf das Magnetfeld.

Es sei an dieser Stelle darauf hingewiesen, dass das Gehäuse 104a,b nicht notwendiger Weise längs der Schwerkraftrichtung ausgerichtet sein muss sondern auch einen Winkel dazu einnehmen kann. In diesem Fall ändert sich das Raum-Koordinatensystem und die Bezugsachsen für "Gieren" und "Nicken" entsprechend.

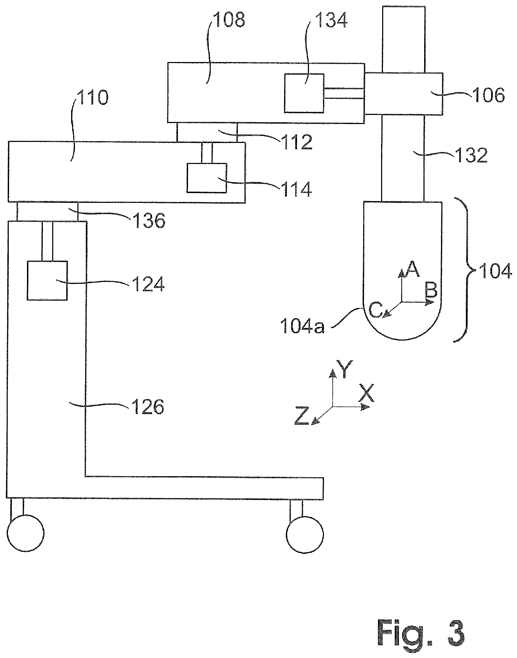

Das Ausführungsbeispiel gemäß der Fig. 3 zeigt eine Positioniervorrichtung 102 (Roboterarmsystem), das sich konstruktiv und auch konzeptionell von der Positioniervorrichtung 102 des ersten und zweiten Ausführungsbeispiels unterscheidet.

In diesem Fall sind die beiden seriell aneinander anscharnierten Schwenkarme 108, 110 mittels eines vertikalen Scharniers 112 miteinander gekoppelt, das lediglich eine horizontale Relativdrehung der beiden Schwenkarme 108, 110 erlaubt. Der eine (freie) Schwenkarm 108 ist an seinem freien Ende ebenfalls mit einem Anschlussinterface 106 versehen, dieses mal jedoch bestehend aus einer Aufnahme für eine Teleskopstange 132, die vertikal innerhalb der Aufnahme 106 motorisch (per Aktuator 134) angetrieben verfahrbar ist. Die Teleskopstange 132 kann hierbei beispielsweise eine Zahnstange, eine Kolbenstange oder dergleichen Schubstange sein, die in der Aufnahme 106 beispielsweise ein Getriebegehäuse, ein Druckzylinder oder dergleichen Antriebseinheit vertikalverschiebbar geführt ist.

Das freie Ende des anderen (eingebundenen) Schwenkarms 110 ist mit einem Sockel der vorzugsweise manuell verfahrbaren Basis 126 über ein vertikales Scharnier 136 gekoppelt. Auch dieses Scharnier 136 ist über den Aktor 124 (motorisch) aktivierbar.

Schließlich ist an einem Ende (das vertikal untere Ende) der Teleskop- oder Schubstange 132 der magnetische Endeffektor 104 angeordnet. Dieser magnetische Endeffektor 104 kann hierbei sowohl nach dem Prinzip des ersten wie auch des zweiten Ausführungsbeispiels gemäß vorstehender Beschreibung aufgebaut sein. D.h. er kann mit einem einzigen Gehäuse oder mit zwei zueinander verdrehbaren Gehäusen ausgebildet sein, wobei im ersten Fall zwei und im zweiten Fall nur ein Freiheitsgrad eingehaust ist.

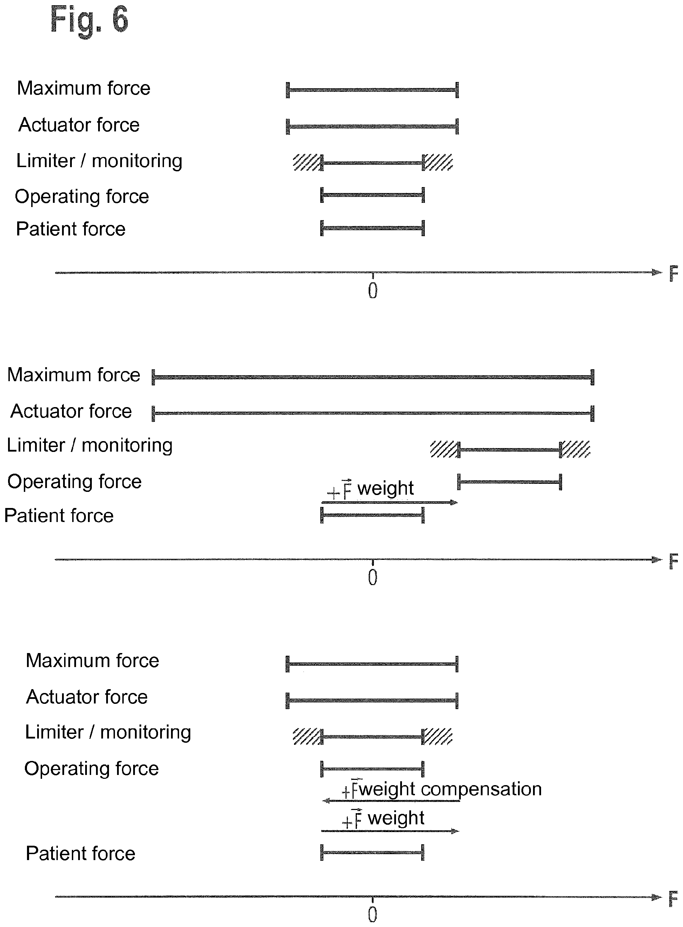

Aus der grundsätzlichen Konzeption der vorstehend beschriebenen Positioniervorrichtungen ergibt sich, dass die Aktorik des ersten und zweiten Ausführungsbeispiels aufgrund der horizontalen Ausrichtung der jeweiligen Scharniere mit dem Eigengewicht der betreffenden Schwenkarme 108, 110 und der daran angeordneten Last (Endeffektor) beaufschlagt werden, wohingegen beim dritten Ausführungsbeispiel gemäß der Fig. 3 (mit Ausnahme des Anschlussinterface 106) diese Belastung oder Kraft von den vertikalen Scharnieren abgefangen wird. D.h. dass im Fall des dritten Ausführungsbeispiels die Aktorik im Wesentlichen nur die Massenträgheit zur Bewegung der Schwenkarme 108, 110 (einschließlich des Endeffektors 104) überwinden muss, während im Fall des ersten und zweiten Ausführungsbeispiels die Aktorik auch die Belastung aus der Schwerkraft der Schwenkarme 108, 110 zu bewältigen hat. Um diese Problematik zu verdeutlichen, sei an dieser Stelle auf die Fig. 6 verwiesen.

Diese zeigt in der mittleren Darstellung die Aktorenkraft, die notwendig ist, um einen Schwenkarm gemäß dem ersten und zweiten Ausführungsbeispiel zu bewegen. Es sei angenommen, dass die Aktorenkraft geringfügig höher ist als die tatsächlich erforderliche Kraft und mittels elektrischer Begrenzer entsprechend eingeregelt wird. Die Aktorenkraft setzt sich dabei zusammen aus der für die Bewegung des Endeffektors beispielsweise zur Druckbeaufschlagung eines Patientenkörpers erforderliche Kraft sowie der darauf einwirkenden Schwerkraft der Last (einschließlich der jeweiligen Schwenkarme).

Im Gegensatz hierzu zeigt die obere Darstellung die Aktorenkraft im Fall des vorstehenden dritten Ausführungsbeispiels. Wie gut zu erkennen ist reduziert sich die Aktorenkraft im Wesentlichen auf die erforderliche Betätigungskraft (mit einem geringfügigen Überschuss) einschließlich der ggf. notwendigen Druckkraft auf einen Patientenkörper, da die Kraft aus der Schwerkraft der Last von den vertikalen Scharnieren abgefangen wird. In diesem Fall kann also die Aktorik der Positionsvorrichtung deutlich schwächer ausgelegt werden als im ersten und zweiten Ausführungsbeispiel, was positiv auf die Sicherheit des gesamten Systems auswirkt.

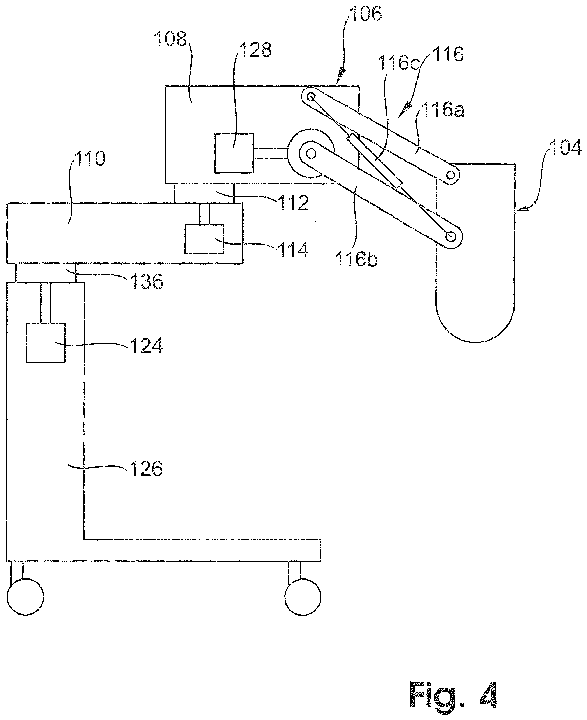

Um die Positioniervorrichtung des ersten und zweiten Ausführungsbeispiels dennoch sicherer und damit für eine medizinische Anwendung tauglich zu machen sieht das vierte Ausführungsbeispiel der Erfindung gemäß der Fig. 4 den Einsatz einer Balancierung (Ausgleichsvorrichtung) vor.

In diesem Fall betrifft die Positioniervorrichtung 102 eine Kombination aus den ersten drei Ausführungsbeispielen, wonach konkret die beiden Schwenkarme 108, 110 der Positioniervorrichtung 102 nach dem Prinzip des dritten Ausführungsbeispiels miteinander gekoppelt und an die verfahrbare Basis 126 anscharniert sind (d.h. mittels vertikaler Scharniere), wohingegen der magnetische Endeffektor 104 über ein horizontales Scharnier 116 mit dem Anschlussinterface 106 der Positioniervorrichtung 102 gekoppelt ist, welches hier jedoch kräfteausgeglichen, d.h. ausbalanciert ist.

Im Einzelnen besteht das horizontale Scharnier 116 aus einem Parallelogrammgelenk mit mindestens zwei parallelen Scharnierarmen 116a, 116b die über eine Diagonalfeder 116c ausbalanciert sind. Die Federkraft ist hierbei auf die Last des magnetischen Endeffektors 104 so abgestimmt, dass die Diagonalfeder 116c die Endeffektorlast im Wesentlichen trägt. Zumindest einer der beiden Scharnierarme 116a, 116b ist ferner über den einen Aktuator 128 betätigbar, um den magnetischen Endeffektor 104 vertikal zu bewegen. Da die Diagonalfeder 128c das Gewicht des Endeffektors 104 im Wesentlichen aufnimmt, muss der Aktuator 128 nur die Massenträgheit zur Bewegung des Endeffektors 104 überwinden und kann dementsprechend leistungsschwach ausgeführt sein. Dies wird beispielsweise in der Fig. 6, untere Darstellung verdeutlicht.

Demzufolge kann die Aktorenkraft mit einem geringen Überschuss im Wesentlichen auf die notwendige Betätigungskraft zur Überwindung der Masseträgheit einschließlich der gewollten Druckkraft auf einen Patientenkörper reduziert werden und entspricht daher weitestgehend der Aktorenkraft gemäß der oberen Darstellung (bezogen auf das dritte Ausführungsbeispiel der Erfindung).

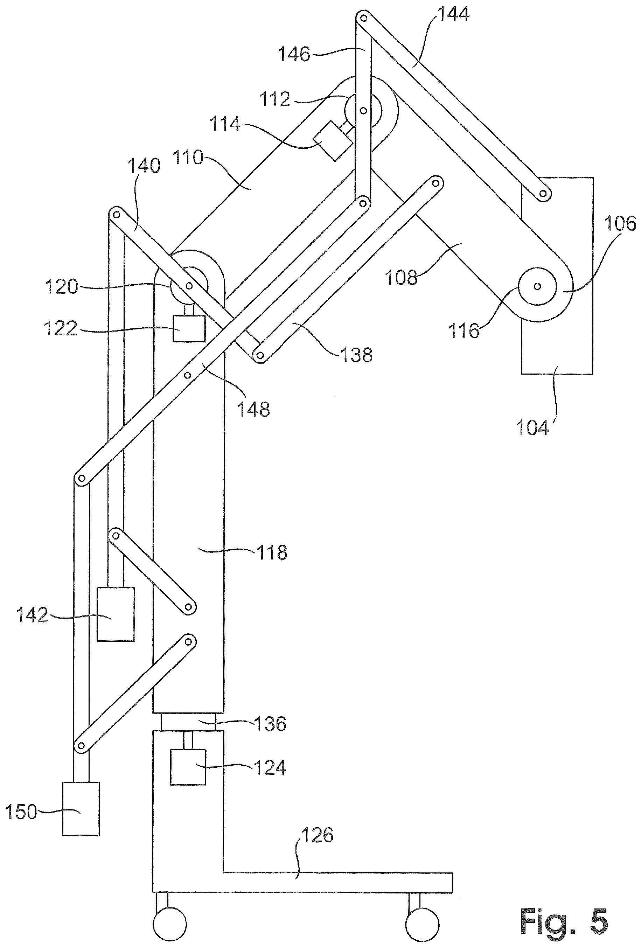

Es liegt auf der Hand, dass das Balancierprinzip auf alle Scharniere der Positioniervorrichtung 102 anwendbar ist, wie dies beispielhaft im fünften Ausführungsbeispiel der Erfindung gemäß der Fig. 5 dargestellt ist.

Der konzeptionelle Aufbau der hier gezeigten Positioniervorrichtung 102 entspricht im Wesentlichen dem ersten oder zweiten Ausführungsbeispiel gemäß der Fig. 1 und 2. In diesem Fall jedoch ist in einem Mittenabschnitt des einen (freien) Schwenkarms 108 eine Schubstange 138 anscharniert, die mit einem Hebelarm 140 gekoppelt ist, der in seinem Mittenabschnitt am Scharnier 120 zwischen dem anderen (eingebundenen) Schwenkarm 110 und dem Rotationsarm 118 angelenkt ist. Am anderen Ende des Hebelarms 140 ist ein Gewicht 142 (über eine vertikal geführte Pendelstange) angeordnet.

Des Weiteren ist eine Zug-/Druckstange 144 mit dem magnetischen Endeffektor 104 vertikal oberhalb des horizontalen Scharniers 116 zwischen Endeffektor 104 und Anschlussinterface 106 angelenkt, die an eine erste Hebelstange 146 gekoppelt ist, die wiederum an dem horizontalen Scharnier 112 zwischen den beiden seriellen Schwenkarmen 108, 110 angelenkt ist. Am anderen Ende der ersten Hebelstange 146 ist eine Zugstange 148 angekoppelt, die in einem Mittenabschnitt des Rotationsarms 118 schwenkbar gelagert ist und am anderen Ende (über eine vertikal geführte Pendelstange) mit einem Gewicht 150 versehen ist. Hierbei sei darauf hingewiesen, dass mit Ausnahme des Scharniers 116 zwischen magnetischen Endeffektor 104 und Anschlussinerface 106 alle anderen Scharniere/Drehachse (motorisch) aktuierbar sind, wohingegen der magnetische Endeffektor 104 über die Zug- und Hebelstangen 14, 146, 148 immer senkrecht ausgerichtet wird. Die vorstehend beschriebene Hebelmechanik sowie die Gewichte sind so ausgelegt, dass die Aktoren unabhängig von der Schwenkposition der Schwenkarme 108, 110 im Wesentlichen kräftefrei sind und daher nur für die Überwindung der Masseträgheit ausgelegt werden müssen.

Zusammenfassend ist an dieser Stelle festzuhalten, dass durch die Unterbringung ausgewählter Freiheitsgrade (Scharniere oder Gelenke) in entsprechenden Gehäusen, vorliegend die Einhausung der Rotationsfreiheitsgrade des Magnetfelderzeugers im Effektorgehäuse, die Zahl der frei zugänglichen Freiheitsgrade (Scharniere) und der daran gekoppelten Schwenkbewegungen von Elementen der magnetischen Führungseinrichtung reduziert werden können, wodurch sich das Verletzungsrisiko einer Bedienerperson und/oder eines Patienten bei Erhaltung der erforderlichen Funktionen verringern lässt. Da darüber hinaus durch die besondere Ausrichtung der Scharniere zwischen den Schwenkarmen (vertikal) und/oder der Ausbalancierung der Scharniere (horizontal) die Aktorik nur für die Überwindung von Trägheitskräften auszulegen ist, können die hierdurch erzielbaren Maximalkräfte soweit verringert werden, dass diese für ein Verletzen einer Bedienerperson oder eines Patienten nicht mehr ausreichen. Der Einsatz einer der beiden genannten Maßnahmen reicht bereits aus, die gestellte Aufgabe zu erfüllen, wobei die Kombination beider Maßnahmen die Sicherheitseigenschaft der magnetischen Führungseinrichtung insgesamt weiter verbessert.

Im Nachfolgenden wird der magnetische Endeffektor gemäß der vorliegenden Erfindung prinzipiell näher beschrieben.

### Magnetischer Endeffektor

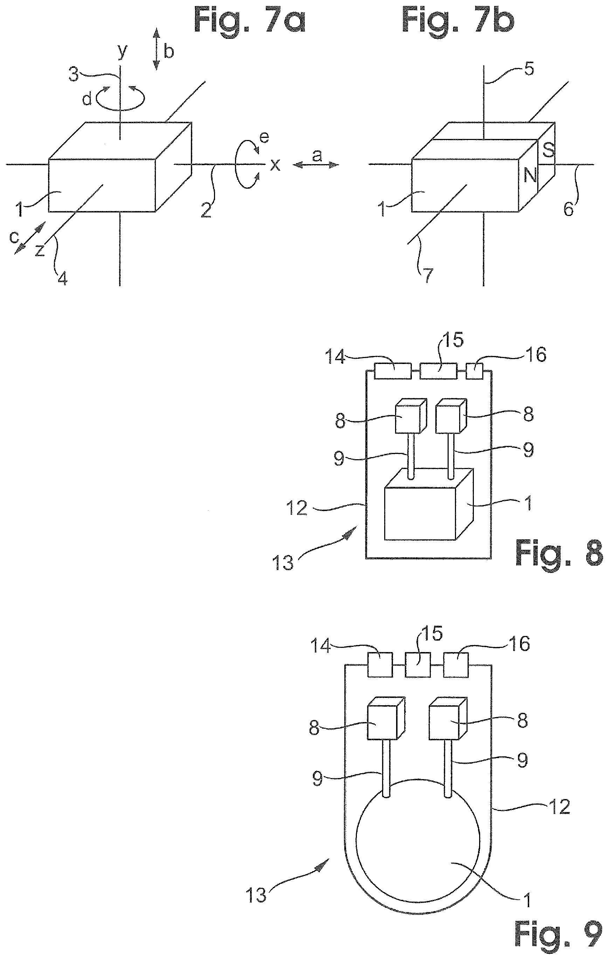

Der magnetische Endeffektor 13 gemäß der Fig. 8 ist eine Vorrichtung, die mindestens einen rotatorischen Freiheitsgrad (Freiheitsgrad d oder e), aber vorzugsweise die beiden rotatorischen Bewegungsfreiheitsgrade (Freiheitsgrad d und e) eines Magnetfelderzeugers 1 gemäß der Fig. 7a und 7b aktuiert. Hierbei handelt es sich vorzugsweise um jeweils eine Rotation des Magnetfelderzeugers 1 in 0-Lage um die Achse A (entspricht in 0-Lage beispielsweise der Y-Achse eines Raum-Koordinatensystems) im Wesentlichen längs der Schwerkraft für ein Schwenken oder Gieren sowie eine Rotation des Magnetfelderzeugers 1 um die Achse B (entspricht beispielsweise der X-Achse des Raum-Koorinatensystems) senkrecht zur Polarisationsachse/Längsachse des Magnetfelderzeugers 1 für ein Nicken. Die Achsen A und B sind vorstehend beschrieben, sodass an dieser Stelle auf die entsprechende Textstelle verwiesen werden kann. Eine Rotation des Magnetfelderzeugers 1 um die Polarisationsachse C (Rollen) scheidet bei dieser Definition gemäß der Fig. 7a aus, da sich hierdurch das Magnetfeld nicht wesentlich ändern würde.

Die Aktuierung der Freiheitsgrade erfolgt derart innerhalb eines Gehäuses 12 des magnetischen Endeffektors 13, dass die Aktuierung nicht zur Änderung der Position, Ausrichtung oder Form des magnetischen Endeffektors 13 (des Gehäuses 12) führt. Vorzugsweise bewegen sich im Ausführungsbeispiel gemäß der Fig. 8 (entspricht dem Endeffektor gemäß Fig. 1) auch keine oberflächlichen Teile oder Komponenten des Gehäuses 12.

Hierzu weist der magnetische Endeffektor 13 mindestens einen Aktuator 8 auf, der jeweils über ein Übertragungselement 9 derart mit dem Magnetfelderzeuger kompakter Bauart 1 gekoppelt ist, dass sich wahlweise eine Aktuierung des Freiheitsgrades d oder eine Aktuierung des Freiheitsgrades e erreichen lässt. Diese Aktuierung macht sich nach außen hin (außerhalb des Gehäuses 12) vorzugsweise nur durch eine per externen Aktuator bewirkbare Drehung des magnetischen Endeffektors bzw. des Gehäuses 12 um den nicht innerhalb des Gehäuses 12 aktuierten Freiheitsgrad und in jedem Fall durch eine sich ändernde Ausrichtung des Magnetfeldes bemerkbar. Dies entspricht dem Endeffektor gemäß der Fig. 2.

In einer vorteilhaften Ausgestaltung des magnetischen Endeffektors 13 gemäß der Fig. 8 und 9 weist der magnetische Endeffektor 13 zwei Aktuatoren 8 auf, die jeweils über ein Übertragungselement 9 derart mit dem Magnetfelderzeuger kompakter Bauart 1 gekoppelt sind, dass sich sowohl eine Aktuierung des Freiheitsgrades d als auch eine Aktuierung des Freiheitsgrades e innerhalb des Gehäuses 12 erreichen lässt. Diese Aktuierung macht sich nach außen hin (außerhalb des Gehäuses 12) nur durch eine Ausrichtung des Magnetfeldes bemerkbar, wohingegen das Gehäuse 12 keine Rotationsbewegung ausführt.

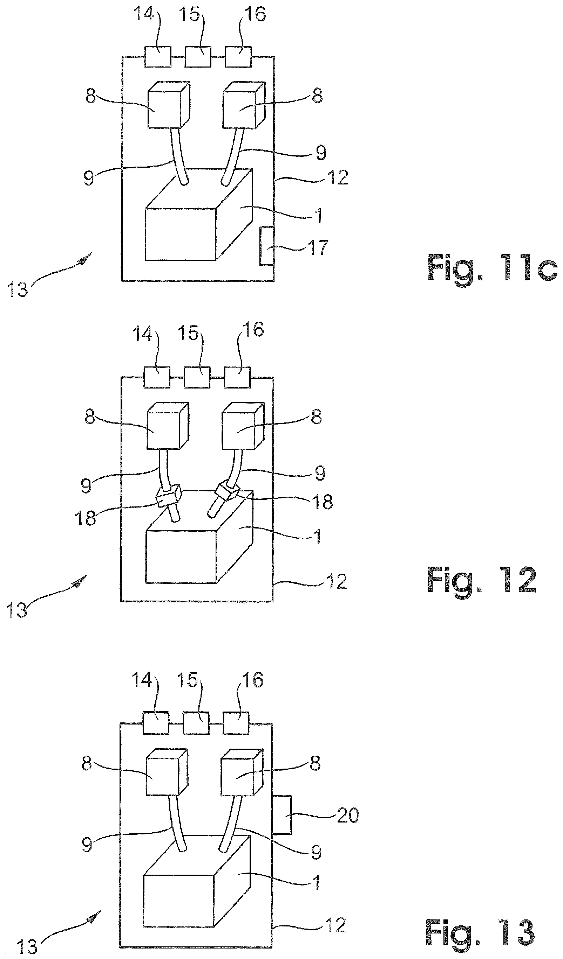

In einer vorteilhaften Ausgestaltung des Übertragungselements 9 gemäß der Fig. 12 verfügt das/jedes Übertragungselement 9 über ein Sensorelement 18, das Informationen erhebt, die Rückschlüsse mindestens auf die Position des Magnetfelderzeugers kompakter Bauart 1 zulässt. Diese Sensorelemente 18 können beispielsweise Bewegungs- Lage- oder Kraftsensoren sein.

In einer vorteilhaften Ausgestaltung des Übertragungselementes 9 verfügt das Übertragungselement 9 somit über ein (weiteres) Sensorelement 18, das Informationen erhebt, die Rückschlüsse mindestens auf die auf den Magnetfelderzeuger kompakter Bauart 1 wirkende Kraft zulässt.

Das wesentliche Merkmal der konstruktiven Auslegung des magnetischen Endeffektors 13 ist, dass sich die Aktuatoren 8 sowie die Übertragungselemente 9 innerhalb des Gehäuses 12 des magnetischen Endeffektors 13 befinden. Der zentrale Vorteil dieser konstruktiven Auslegung des magnetischen Endeffektors 13 ist, dass die Aktuierung der Freiheitsgrade d und/oder e des Magnetfelderzeugers kompakter Bauart 1 zu keinen für die Patientensicherheit relevanten Bewegungen des magnetischen Endeffektors 13 führt.

Der magnetische Endeffektor 13 verfügt über ein Verbindungselement 14 zur Übertragung von Kräften. Das Verbindungselement 14 dient zur Ausrichtung des magnetischen Endeffektors 13 im Raum (vorzugsweise in Vertikalrichtung) durch Einleitung von Haltekräften bzw. Kräften zur Beschleunigung des magnetischen Endeffektors 13.

Der magnetische Endeffektor 13 verfügt ferner über ein Verbindungselement 15 zur Übertragung von Energie. Diese Energie wird zumindest zum Betrieb der Aktuatoren 8 verwendet. In einer vorteilhaften Ausgestaltung des Verbindungselements 15 wird über das Verbindungselement 15 vorzugsweise elektrische Energie übertragen. Diese Ausgestaltung hat den Vorteil, dass sich mit der elektrischen Energie neben den Aktuatoren 8 auch im magnetischen Endeffektor vorhandene elektronische Komponenten betreiben lassen. In einer weiteren vorteilhaften Ausgestaltung des Verbindungselements 15 wird über das Verbindungselement 15 vorzugsweise pneumatische oder hydraulische Energie übertragen.

Der magnetische Endeffektor 13 verfügt schließlich über ein Verbindungselement 16 zur Übertragung von Daten. Sämtliche genannten Verbindungselemente 14, 15, 16 sind mit einem Anschlussinterface der Positioniervorrichtung verbindbar bzw. verbunden.

In einem vorteilhaften Anwendungsszenario wird der magnetische Endeffektor 13 nur oberhalb des vorzugsweise liegenden Patientenkörpers geführt. In Verbindung mit dem in der EP 2 347 699 A1 offenbarten Steuerungsprinzip eines intrakorporalen magnetischen Objektes ist der magnetische Endeffektor 13 immer oberhalb eines zu steuernden intrakorporalen Objekts (nicht weiter dargestellt) zu positionieren. Damit weist die als Kontaktseite vorgesehene Unterseite des magnetischen Endeffektors 13 vorzugsweise in Richtung des Patienten, d.h. nach unten.

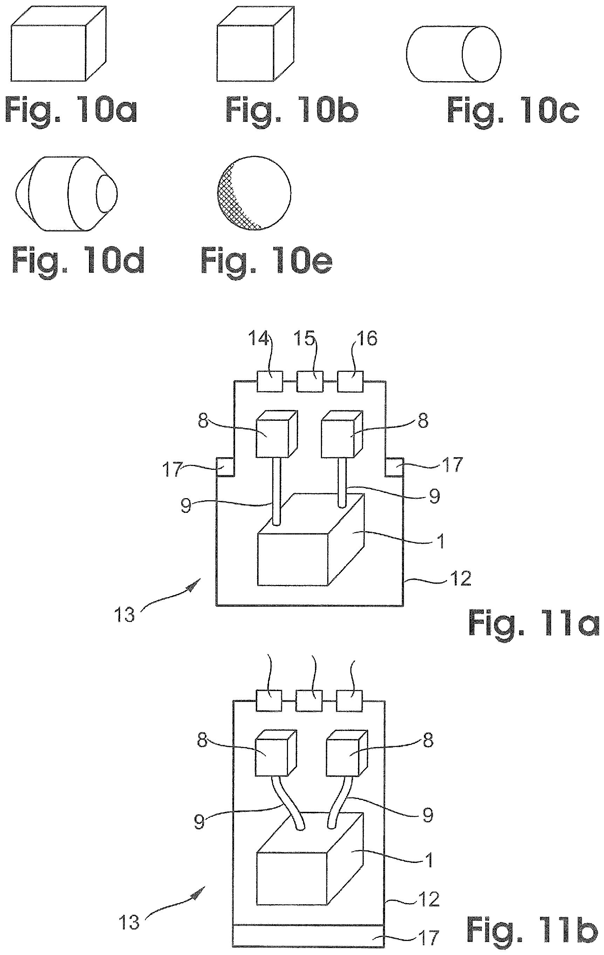

In einer vorteilhaften Ausgestaltung des magnetischen Endeffektors 13 weist die dem Patientenkörper vorzugsweise zugewandte Unterseite des Gehäuses 12 daher eine Rundung auf, die vorzugsweise als hemisphärische Form ausgebildet ist. Des Weiteren ist gemäß derselben vorteilhaften Ausgestaltung des magnetischen Endeffektors 13 der Magnetfelderzeuger kompakter Bauart 1 in einer gedrungenen Form ausgebildet. In einer vorteilhaften Ausgestaltung des Magnetfelderzeugers kompakter Bauart 1 mit gedrungener Form ist dieser als Quader (Fig. 10a) ausgebildet. In einer anderen vorteilhaften Ausgestaltung des Magnetfelderzeugers kompakter Bauart 1 mit gedrungener Form ist dieser als Würfel (Fig. 10b) ausgebildet. In einer anderen vorteilhaften Ausgestaltung des Magnetfelderzeugers kompakter Bauart 1 mit gedrungener Form ist dieser als Zylinder (Fig. 10c) ausgebildet. In einer anderen vorteilhaften Ausgestaltung des Magnetfelderzeugers kompakter Bauart 1 mit gedrungener Form ist dieser als Zylinder mit gefasten oder abgerundeten Kanten ausgebildet (Fig. 10d). In einer anderen vorteilhaften Ausgestaltung des Magnetfelderzeugers kompakter Bauart 1 mit gedrungener Form ist dieser als Kugel ausgebildet (Fig. 10e). In einer anderen vorteilhaften Ausgestaltung des Magnetfelderzeugers kompakter Bauart 1 mit gedrungener Form ist dieser als jede sonstige geometrische Form ausgebildet, die einer gedrungenen Bauweise zuträglich ist.

Der Vorteil dieser vorteilhaften Ausgestaltungen des magnetischen Endeffektors 13 ist, dass sich durch Abrundung der dem Patientenkörper vorzugsweise zugewandten Unterseite des Gehäuses 12 und Einpassung des Magnetfelderzeugers kompakter Bauweise 1 in die Form der Unterseite des Gehäuses 12 durch Ausgestaltung des Magnetfelderzeugers 1 in gedrungener Form sich der Magnetfelderzeuger 1 so nah wie möglich an das zu steuernde intrakorporale Objekt approximieren lässt, während gleichzeitig die Aktuierung der zwei Freiheitsgrade d und e innerhalb des Gehäuses 12 nicht beeinträchtig wird. Dies gilt insbesondere für Manöver, bei denen der magnetische Endeffektor 13 mit definierter Kraft auf den Patientenkörper aufgedrückt wird, um sich möglichst nah an das zu steuernde intrakorporale Objekt zu positionieren.

In einer vorteilhaften Ausgestaltung des Gehäuses 12 gemäß der Fig. 11a - 11c weist das Gehäuse 12 mindestens ein Sensorelement 17 auf. Dieses zumindest eine Sensorelement 17 wird zur Erkennung von durch Kollision aufgetretenen Kräften auf das Gehäuse 12 verwendet.

In einer vorteilhaften Ausgestaltung des zumindest einen Sensorelements 17 ist dieses Sensorelement 17 ein Deformationssensor und derart mit dem Gehäuse 12 verbunden, dass es in einem abgegrenzten Bereich des Gehäuses 12 Deformationen registrieren kann und damit ein Rückschluss auf die in das Gehäuse eingeleitete Kraft gezogen werden kann.

In einer vorteilhaften Ausgestaltung des zumindest einen Sensorelements 17 ist dieses Sensorelement 17 ein Kraftsensor und derart mit dem Gehäuse 12 verbunden, dass es als Verbindungselement zwischen zwei Elementen des Gehäuses 12 eine von außen eingeleitete Kraft auf eines der Elemente registrieren kann.

In einer vorteilhaften Ausgestaltung des zumindest einen Sensorelements 17 ist dieses Sensorelement 17 ein mechanischer Schalter und derart mit dem Gehäuse 12 verbunden, dass es als Verbindungselement zwischen zwei Elementen des Gehäuses 12 fungiert und eine von außen eingeleitete Kraft auf eines der Elemente zu einer Betätigung des Sensorelements 17 führt.

In einer vorteilhaften Ausgestaltung des zumindest einen Sensorelements 17 ist dieses Sensorelement 17 ein kraftempfindliches Element, das an der Außenseite des Gehäuses 12 derart angebracht ist, dass Kontaktkräfte auf das Sensorelement 17 registriert werden.

In einer vorteilhaften Ausgestaltung des Gehäuses 12 gemäß der Fig. 13 weist das Gehäuse 12 (zusätzlich zu den genannten Sensoren) ein Bedienelement 20 auf.

In einer vorteilhaften Ausgestaltung des Bedienelements 20 weist das Bedienelement 20 mindestens einen Griff auf, der eine manuelle Betätigung des magnetischen Endeffektors 13 unter Umgehung/Überwindung der Aktorik der Positionsvorrichtung ermöglicht.

In einer weiteren vorteilhaften Ausgestaltung des Bedienelements 20 weist das Bedienelement 20 mindestens einen berührungsempfindlichen Schalter zur manuellen Aktivierung einzelner Aktoren der Positioniervorrichtung auf.

In einer weiteren vorteilhaften Ausgestaltung des Bedienelements 20 weist das Bedienelement 20 mindestens ein optisches Anzeigeelement beispielsweise zur Darstellung von virtuellen Bewegungsbarrieren der Positioniervorrichtung gemäß vorstehender Beschreibungseinleitung auf.

### Positioniervorrichtung zur Ausrichtung des magnetischen Endeffektors gemäß vorstehender Beschreibung.

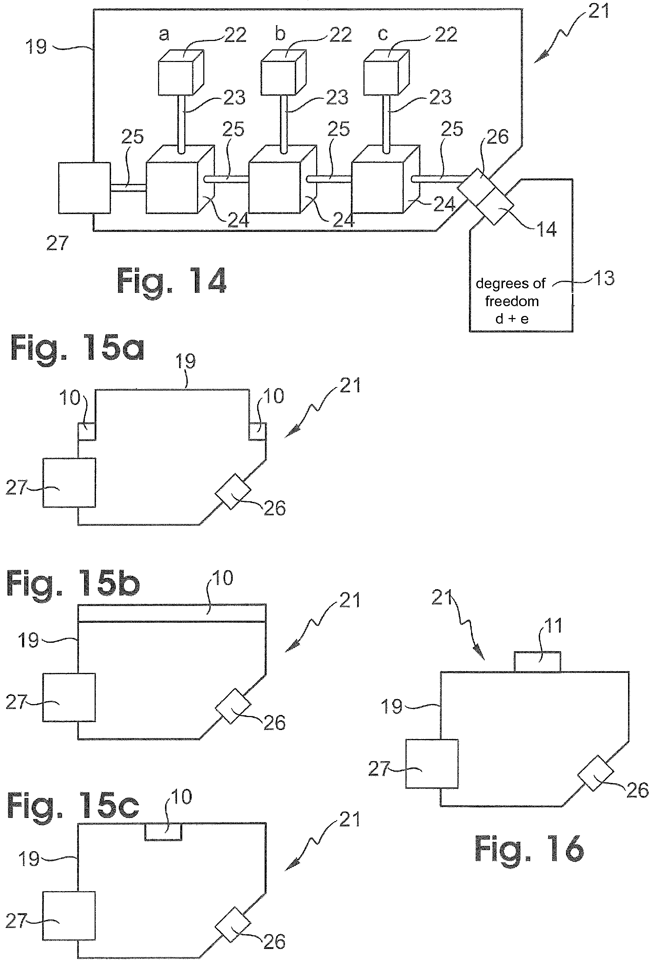

Die Positioniervorrichtung 21 zur Ausrichtung des magnetischen Endeffektors 13 gemäß der Fig. 14 (siehe Systemlinie 21 in Fig. 14) hat die Aufgabe, den magnetischen Endeffektor 13 im Raum (Raum-Koordinatensystem) zu positionieren. Der magnetische Endeffektor 13 nach oben beschriebener vorzugsweisen Auslegung ist in der Lage, die Freiheitsgrade d und e des Magnetfelderzeugers kompakter Bauart 1 zu steuern. Die Aufgabe der Positioniervorrichtung 21 ist es folglich, den magnetischen Endeffektor 13 entlang der Freiheitsgrade a, b und c zu positionieren. So lässt sich durch die Kombination aus Positioniervorrichtung 21 und magnetischem Endeffektor 13 (bilden gemeinsam die Führungseinrichtung) der Magnetfelderzeuger kompakter Bauart 1 in allen für die Steuerung intrakorporaler Objekte erforderlichen (sinnvollen) fünf Freiheitsgrade a bis e ausrichten.

Die Positioniervorrichtung 21 bewegt den magnetischen Endeffektor 13 (translatorisch) im Raum. Diese Bewegungen können zur Kollision mit und Krafteinwirkung auf den Patientenkörper führen, wie dies eingangs bereits beschrieben wurde, wobei bestimmte Kollisionen oder Krafteinwirkungen ggf. sogar beabsichtigt sind. Die konstruktive Auslegung der Positioniervorrichtung 21 sowie die Aktuierung derselben sind daher erfindungsgemäß derart gestaltet, dass eine Kollision mit und Krafteinwirkung auf den Patientenkörper nicht zu den Patienten gefährdenden Einwirkungen auf den Patientenkörper kommen kann. Vorzugsweise ist die konstruktive Auslegung der Positioniervorrichtung 21 sowie die Aktuierung derselben erfindungsgemäß derart gestaltet, dass es selbst bei Fehlbedienung oder Fehlfunktion einer oder mehrerer Komponenten der Positioniervorrichtung nicht zu den Patienten gefährdenden Einwirkungen auf den Patientenkörper kommen kann.

Die Positioniervorrichtung 21 verfügt über mindestens drei Aktuatoren 22, wie dies bereits anhand der Fig. 1 bis 5 beschrieben wurde, die jeweils über ein Übertragungselement 23 mit jeweils einem Gelenk (Scharnier in horizontaler oder vertikaler Ausrichtung) 24 in Verbindung stehen. Der Begriff "Gelenk" umfasst sowohl rotatorische Gelenke/Scharniere, aber auch Linearführungen (Teleskopstangen, Zahnstangen, Kolben-/Zylinderanordnungen, etc.) oder eine Reihe von Kettengliedern. Die Konstruktion ist derart gestaltet, dass ein Aktuator 22 mit dem jeweiligen Gelenk 24 über das jeweilige Übertragungselement 23 derart in Verbindung steht, dass der Aktuator 22 das jeweilige Gelenk 24 betätigen kann.

Der Aktuator 22 kann als Rotationsaktuator beispielsweise nach der Bauart eines Elektromotors, oder als Linearaktuator beispielsweise nach der Bauart eines fluidischen Hubzylinders ausgestaltet sein.

Die Positioniervorrichtung 21 weist ein Verbindungselement 26 (entspricht dem beschriebenen Anschlussinterface enthaltend die Anschlüsse für die Verbindungselemente 14 bis 16 des Endeffektors 13) zur Übertragung von Kräften und Energie sowie zur mechanischen Kopplung der Positioniervorrichtung 21 mit dem magnetischen Endeffektor 13 geeignet ist.

Die Positioniervorrichtung 21 weist zudem ein Befestigungselement 27 auf, das zur ortsfesten Fixierung der Positioniervorrichtung 21 im Raum dient. In einer vorteilhaften Ausgestaltung des Befestigungselements 27 ist das Befestigungselement 27 als die Konsole oder Basis gemäß der Fig. 1 bis 5 ausgeformt, die sich beispielsweise durch arretierbare Rollen wahlweise im Raum arretieren oder verschieben lässt. In einer weiteren vorteilhaften Ausgestaltung des Befestigungselements 27 weist das Befestigungselement 27 mindestens eine Vorrichtung auf, die sich zur Fixierung an einem Gegenstand, beispielsweise dem Untersuchungsbett, eignet.

Die Positioniervorrichtung 21 weist zudem Verbindungselemente 25 auf, die eine mechanische Verbindung mit einem Gelenk 24 (entspricht dem Rotationsarm gemäß der Fig. 1 bis 5) und beispielsweise einem weiteren Gelenk 24 (entspricht dem Scharnier zwischen Rotationsarm und eingebundenem Schwenkarm gemäß der Fig. 1 bis 5), dem Verbindungselement 26 (entspricht dem Anschlussinterface) oder dem Befestigungselement 27 (entspricht der verfahrbaren Basis gemäß der Fig. 1 bis 5) herstellt. Ein Verbindungselement 25 kann beispielsweise eine Montageplatte sein, mit dem das eine Gelenk 24 und beispielsweise das weitere Gelenk 24, das Verbindungselement 26 oder das Befestigungselement 27 kraftschlüssig fixiert ist.

Die einzelnen Gelenke (Scharniere/Rotationsachse) 24 sowie das Verbindungselement 26 und das Befestigungselement 27 sind über mechanische Verbindungselemente 25 (Schwenkarme/Rotationsarm) derart miteinander verbunden, dass sich durch Betätigung der Gelenke 24 das Verbindungselement 26 relativ zum Befestigungselement 27 zumindest entlang der drei zueinander rechtwinkligen Raumachsen positionieren lässt.

Grundsätzlich besteht bei der erfindungsgemäßen Positioniervorrichtung 21 die Möglichkeit ausgewählte oder alle Aktuator 22 - Verbindungselement 23 - Gelenk 24 - Einheiten mit Sensoren auszurüsten, welche Sicherheitsfunktionen für die Positioniervorrichtung 21 ausüben.

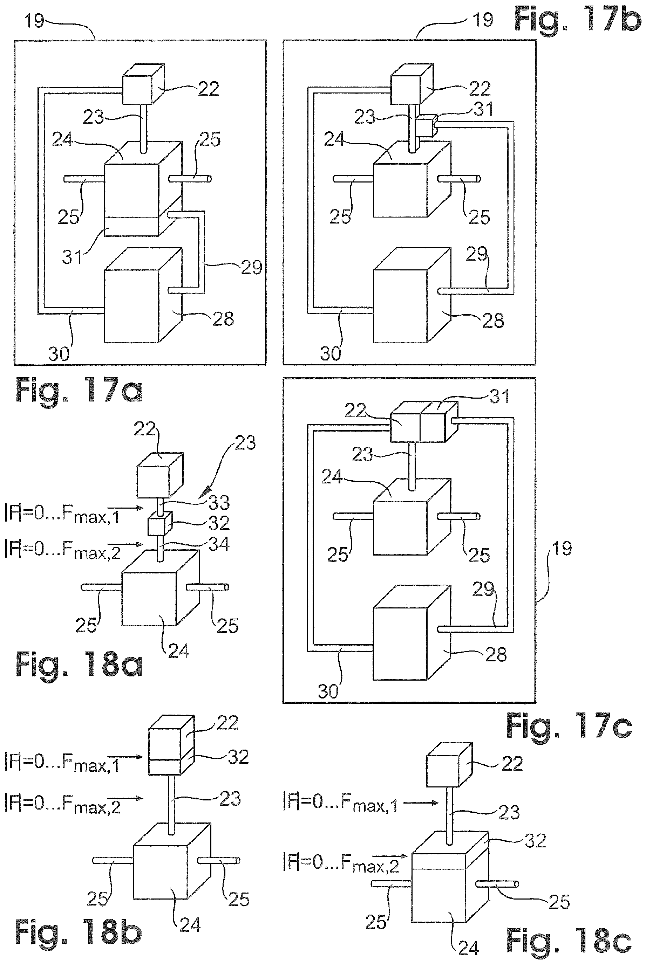

In einer vorteilhaften Ausgestaltung eines (oder mehrerer) ausgewählten Aktuators 22 weist der Aktuator 22 (wie prinzipiell in der Fig. 18b gezeigt ist) ein Sensorelement auf, das dazu in der Lage ist, die zu übertragende Kraft zu messen. In einer weiteren vorteilhaften Ausgestaltung des Aktuators 22 weist der Aktuator 22 eine Vorrichtung auf (siehe Fig. 17c), die dazu in der Lage ist, die vom Aktuator 22 zur Krafterzeugung aufgewandte Energie zu messen. In einer weiteren vorteilhaften Ausgestaltung des Aktuators 22 weist der Aktuator 22 eine Vorrichtung auf (siehe Fig. 17c), die dazu in der Lage ist, die zu übertragende Kraft mechanisch zu begrenzen. In einer weiteren vorteilhaften Ausgestaltung des Aktuators 22 weist der Aktuator 22 eine Vorrichtung (nicht weiter dargestellte Endanschläge) auf, die dazu in der Lage ist, den Bewegungsraum des Aktuators 22 mechanisch zu begrenzen. In einer weiteren vorteilhaften Ausgestaltung des Aktuators 22 weist der Aktuator 22 eine nicht weiter gezeigte Vorrichtung auf, die dazu in der Lage ist, eine konstante Betätigungskraft auf das Betätigungselement des Aktuators 22 auszuüben.

In einer vorteilhaften Ausgestaltung des Übertragungselements 23 weist das Übertragungselement 23 eine Vorrichtung auf (siehe Fig. 18a und 18b), die dazu in der Lage ist, die zu übertragende Kraft mechanisch zu begrenzen. In einer weiteren vorteilhaften Ausgestaltung des Übertragungselements 23 weist das Übertragungselement 23 eine Vorrichtung auf (nicht weiter dargestellte Endanschläge), die dazu in der Lage ist, den Bewegungsraum des Übertragungselements 23 mechanisch zu begrenzen. In einer weiteren vorteilhaften Ausgestaltung des Übertragungselements 23 weist das Übertragungselement 23 ein Sensorelement auf, das dazu in der Lage ist, die zu übertragende Kraft zu messen. In einer weiteren vorteilhaften Ausgestaltung des Übertragungselements 23 weist das Übertragungselement 23 eine Vorrichtung auf, die dazu in der Lage ist, eine konstante Betätigungskraft auf das Übertragungselement 23 auszuüben.

In einer vorteilhaften Ausgestaltung des Gelenks 24 weist das Gelenk 24 eine Vorrichtung auf (siehe Fig. 17a und 18c), die dazu in der Lage ist, die Betätigungskraft des Gelenks 24 mechanisch zu begrenzen. In einer weiteren vorteilhaften Ausgestaltung des Gelenks 24 weist das Gelenk 24 eine Vorrichtung auf (nicht weiter dargestellte Endanschläge), die dazu in der Lage ist, den Bewegungsraum des Gelenks 24 mechanisch zu begrenzen. In einer weiteren vorteilhaften Ausgestaltung des Gelenks 24 weist das Gelenk 24 ein Sensorelement auf (siehe Fig. 17a), das dazu in der Lage ist, die Betätigungskraft des Gelenks 24 zu messen. In einer weiteren vorteilhaften Ausgestaltung des Gelenks 24 weist das Gelenk 24 eine Vorrichtung auf, die dazu in der Lage ist, eine konstante Betätigungskraft auf das Gelenk 24 auszuüben.

In einer vorteilhaften Ausgestaltung der Positioniervorrichtung 21 weist die Positioniervorrichtung 21 ein oder mehrere relativ zueinander bewegbar gelagerte Gehäuse 19 auf.

In einer vorteilhaften Ausgestaltung jedes Gehäuses 19 weist das Gehäuse 19 mindestens ein Sensorelement 10 auf (siehe hierzu insbesondere Fig. 15a bis 15c). Jedes Sensorelement 10 wird zur Erkennung von durch Kollision aufgetretenen Kräften auf das Gehäuse 19 verwendet.

In einer vorteilhaften Ausgestaltung des Sensorelements 10 ist dieses Sensorelement 10 ein Deformationssensor und derart mit dem Gehäuse 19 verbunden, dass es in einem abgegrenzten Bereich des Gehäuses 19 Deformationen registrieren kann und damit ein Rückschluss auf die in das Gehäuse eingeleitete Kraft gezogen werden kann.

In einer vorteilhaften Ausgestaltung des Sensorelements 10 ist dieses Sensorelement 10 ein Kraftsensor und derart mit dem (jedem) Gehäuse 19 verbunden, dass es als Verbindungselement zwischen zwei Elementen des Gehäuses 19 eine von außen eingeleitete Kraft auf eines der Elemente registrieren kann.

In einer vorteilhaften Ausgestaltung des Sensorelements 10 ist dieses Sensorelement 10 ein mechanischer Schalter und derart mit dem (jedem) Gehäuse 19 verbunden, dass es als Verbindungselement zwischen zwei Elementen des Gehäuses 19 fungiert und eine von außen eingeleitete Kraft auf eines der Elemente zu einer Betätigung des Sensorelements 10 führt.

In einer vorteilhaften Ausgestaltung des Sensorelements 10 ist dieses Sensorelement 10 ein kraftempfindliches Element, das an der Außenseite des (jedes) Gehäuses 19 derart angebracht ist, dass Kontaktkräfte auf das Sensorelement 10 registriert werden.

In einer vorteilhaften Ausgestaltung des Gehäuses 19 weist das Gehäuse 19 ein Bedienelement 11 auf.

In einer vorteilhaften Ausgestaltung des Bedienelements 11 weist das Bedienelement 11 mindestens einen Griff auf, der die Betätigung Positioniervorrichtung 21 ermöglicht.

In einer weiteren vorteilhaften Ausgestaltung des Bedienelements 11 weist das Bedienelement 11 mindestens einen berührungsempfindlichen Schalter beispielsweise zur manuellen Betätigung eines entsprechend eingehausten Aktuators 22 auf.

In einer weiteren vorteilhaften Ausgestaltung des Bedienelements 11 weist das Bedienelement 11 mindestens ein optisches Anzeigeelement auf.

### Technische Maßnahmen zur Gewährleistung der Patientensicherheit.

Patientenrisiken durch die robotisch geführte Ausrichtung des Magnetfelderzeugers kompakter Bauart 1 ergeben sich unter anderem durch die Bewegungen der Positioniervorrichtung 21 zur Ausrichtung des Magnetfelderzeugers kompakter Bauart 1, die zu Kollisionen mit oder Krafteinwirkung auf den Patientenkörper und damit zu den Patienten gefährdenden Einwirkungen auf den Patientenkörper führen können. Die nachfolgende Anzahl erfindungsgemäßer technischer Maßnahmen eignen sich besonders zur Gewährleistung der Patientensicherheit in diesem Sinne:
- eine Überwachungsvorrichtung 31 zur Überwachung von Position und auftretenden Kräften, wie sie insgesamt in den Fig. 17a -17c und 18a - 18c gezeigt ist,
- die Positioniervorrichtung 21, die dazu in der Lage ist, Betriebszustände der Positioniervorrichtung 21, insbesondere den Patienten gefährdende Betriebszustände der Positioniervorrichtung 21, zu erkennen, anzuzeigen oder zu vermeiden, vorzugsweise in Verbindung mit einer Regelungseinrichtung 28,
- eine Positionsbegrenzungsvorrichtung, die dazu in der Lage ist, den Bewegungsraum der Positioniervorrichtung 21 (mechanisch oder virtuell) zu begrenzen, sowie
- eine Kraftbegrenzungsvorrichtung, die dazu in der Lage ist, die an der Positioniervorrichtung 21 auftretenden Kräfte zu begrenzen.

Eine Kollision der Positioniervorrichtung 21 mit oder Krafteinwirkung der Positioniervorrichtung 21 auf den Patientenkörper kann mittels der Überwachungsvorrichtung 31 erkannt werden. Eine solche Überwachungseinrichtung 31 kann beispielsweise ein Sensorelement sein, das dazu in der Lage ist, die in der Positioniervorrichtung 21 auftretenden oder auf die Positioniervorrichtung 21 wirkenden Kräfte zu messen.

Eine vorteilhafte Ausgestaltung der Überwachungsvorrichtung 31 ist ein Sensorelement 10, beispielsweise in einer der genannten vorteilhaften Ausgestaltungen. Damit ist die Überwachungsvorrichtung 31 in der Lage, die auf das Gehäuse 19 der Positioniervorrichtung 21 einwirkenden Kräfte zu messen und so Rückschlüsse auf die auf einen Patientenkörper wirkenden Kräfte im Falle einer Kollision zuzulassen.

In einer weiteren vorteilhaften Ausgestaltung der Überwachungsvorrichtung 31 ist die Überwachungsvorrichtung 31 ein Sensorelement, das dazu in der Lage ist, die im Gelenk 24 wirkenden Kräfte zu messen.

In einer weiteren vorteilhaften Ausgestaltung der Überwachungsvorrichtung 31 ist die Überwachungsvorrichtung 31 ein Sensorelement, das dazu in der Lage ist, die Position des Gelenks 24 zu messen.

In einer weiteren vorteilhaften Ausgestaltung der Überwachungsvorrichtung 31 ist die Überwachungsvorrichtung 31 ein Sensorelement, das dazu in der Lage ist, die im Übertragungselement 23 wirkenden Kräfte zu messen.

In einer weiteren vorteilhaften Ausgestaltung der Überwachungsvorrichtung 31 ist die Überwachungsvorrichtung 31 ein Sensorelement, das dazu in der Lage ist, die Position des Übertragungselements 23 zu messen.

In einer weiteren vorteilhaften Ausgestaltung der Überwachungsvorrichtung 31 ist die Überwachungsvorrichtung 31 ein Sensorelement, das dazu in der Lage ist, die im Aktuator 22 auftretenden Kräfte zu messen.

In einer weiteren vorteilhaften Ausgestaltung der Überwachungsvorrichtung 31 ist die Überwachungsvorrichtung 31 eine Vorrichtung, die dazu in der Lage ist, die vom Aktuator 22 zur Krafterzeugung aufgewandte Energie zu messen.

In einer weiteren vorteilhaften Ausgestaltung der Überwachungsvorrichtung 31 ist die Überwachungsvorrichtung 31 ein Sensorelement, das dazu in der Lage ist, die Position des Aktuators 22 zu messen.

In einer vorteilhaften Ausgestaltung der Überwachungsvorrichtung 31 wird die Überwachungsvorrichtung 31 in Verbindung mit einer Regelungseinrichtung 28 betrieben. Die Regelungseinrichtung 28 ist über ein Übertragungselement 29 für Daten mit der Überwachungsvorrichtung 31 derart verbunden, dass Daten über den Zustand des von der Überwachungsvorrichtung 31 zu überwachenden Parameters von der Überwachungsvorrichtung 31 zu der Regelungseinrichtung 28 übertragen werden. Des Weiteren ist die Regelungseinrichtung 28 über ein Übertragungselement 30 für Daten direkt oder indirekt mit dem Aktuator 22 derart verbunden, dass die Regelungseinrichtung 28 dazu in der Lage ist, den Betriebszustand des Aktuators 22 zu beeinflussen. Die Rückkoppelung der von der Überwachungsvorrichtung 31 zu überwachenden Parameter über eine Regelungseinrichtung 31 zu dem Aktuator 22 bildet einen geschlossenen Regelkreis. Diese vorteilhafte Ausgestaltung ermöglicht es der Positioniervorrichtung 21, den Patienten gefährdende Krafteinwirkungen auf den Patientenkörper nicht nur zu erkennen, sondern wahlweise auch zu vermeiden.

Die Regelungseinrichtung 28 kann sich wahlweise innerhalb oder außerhalb des Gehäuses 19 der Positioniervorrichtung 21 befinden.

Für den Fall, dass das Risiko von den Patienten gefährdenden Einwirkungen durch eine Kollision der Positioniervorrichtung 21 mit oder Krafteinwirkung der Positioniervorrichtung 21 auf den Patientenkörper mit Bereichen des Bewegungsraums der Positioniervorrichtung 21 in Verbindung gebracht werden kann, kann mittels einer Positionsbegrenzungsvorrichtung das Risiko einer Kollision der Positioniervorrichtung 21 mit oder Krafteinwirkung der Positioniervorrichtung 21 auf den Patientenkörper in bestimmten Bereichen des Bewegungsraums der Positioniervorrichtung 21 ausgeschlossen werden. Eine solche Positionsbegrenzungsvorrichtung kann beispielsweise ein mechanischer Anschlag sein, der dazu in der Lage ist, den Bewegungsbereich eines Gelenkes 24 mechanisch zu begrenzen oder es handelt sich um einen frei programmierbaren virtuellen Anschlag bei Erreichen einer definierten Raumlage.

Bei einer Kollision der Positioniervorrichtung 21 mit oder Krafteinwirkung der Positioniervorrichtung 21 auf den Patientenkörper kann mittels einer Kraftbegrenzungsvorrichtung 32 die auf den Patientenkörper wirkende Kraft begrenzt werden. Eine solche Kraftbegrenzungsvorrichtung 32 kann beispielsweise eine Rutschkupplung bekannter Bauart sein, die ein Drehmoment von einer ersten Achse nur bis zu einem definierten Maximalwert auf eine zweite Achse übertragen kann. Vorzugsweise ist die Kraftbegrenzungsvorrichtung 32 derart ausgestaltet, dass die Kraftbegrenzung durch eine konstruktive Auslegung der Kraftbegrenzungsvorrichtung 32 erreicht wird.

In einer vorteilhaften Ausgestaltung der Kraftbegrenzungsvorrichtung 32 ist die Kraftbegrenzungsvorrichtung 32 jedoch eine Rutschkupplung, die derart mit dem Übertragungselement 23 verbunden ist, dass eine Kraft, die vom Aktuator 22 auf ein erstes Element 33 des Übertragungselements 23 eingebracht wird, nur bis zu einer definierten Maximalkraft auf ein zweites Element 34 des Übertragungselements 23 eingebracht wird.

In einer weiteren vorteilhaften Ausgestaltung der Kraftbegrenzungsvorrichtung 32 umfasst das Übertragungselement 23 einen Kraftübertragungsmechanismus unter Zuhilfenahme eines Zahnriemens oder Keilriemens, wobei die Kraftbegrenzungsvorrichtung 32 eine Zahnriemenscheibe oder Keilriemenscheibe ist, die eine Vorrichtung aufweist, die in der Lage ist, die auf die Zahnriemenscheibe oder Keilriemenscheibe aufgebrachte Kraft nur bis zu einer definierten Maximalkraft auf einen Abnehmer zu übertragen, wobei der Abnehmer beispielsweise eine Welle, ein Zahnriemen oder Keilriemen ist.

In einer weiteren vorteilhaften Ausgestaltung der Kraftbegrenzungsvorrichtung 32 ist die Kraftbegrenzungsvorrichtung 32 ein integraler Bestandteil des Aktuators 22, der dazu in der Lage ist, die vom Aktuator 22 erzeugte Kraft auf einen definierten Maximalwert zu begrenzen. Es besteht beispielsweise die Möglichkeit, die Kraftbegrenzungseinrichtung 32 im Aktuator durch Verwendung eines Schrittmotors als Aktuator zu integrieren, der konstruktionsbedingt bei Überschreiten einer vorbestimmten Kraft (Drehmoment) außer Tritt fällt und somit die Antriebskraft quasi zu Null wird.

In einer weiteren vorteilhaften Ausgestaltung der Kraftbegrenzungsvorrichtung 32 ist die Kraftbegrenzungsvorrichtung 32 ein integraler Bestandteil des Gelenks 24, die dazu in der Lage ist, die vom Gelenk 24 auf das mechanische Verbindungselement 25 aufgebrachte Kraft auf einen definierten Maximalwert zu begrenzen.

Eine Betätigungsvorrichtung 35 (entspricht der vorstehend genannten Aktuator 22 - Verbindungselement 23 - Gelenk 24 - Einheit) beinhaltet zumindest einen Aktuator 22, zumindest ein Übertragungselement 23, zumindest ein Gelenk 24 sowie zumindest ein mechanisches Verbindungselement 25, (siehe beispielsweise Figur 17a). Eine solche Betätigungsvorrichtung 35 kann als eine Funktionseinheit mit mindestens diesen Komponenten angesehen werden.

In einer vorteilhaften Weiterbildung der Betätigungsvorrichtung 35 besteht das mechanische Verbindungselement 25 aus einem ersten Verbindungselement 41 und einem zweiten Verbindungselement 42, wobei sowohl das erste Verbindungselement 41 als auch das zweite Verbindungselement 42 derart mit dem Gelenk 24 gekoppelt ist, dass zwischen dem ersten Verbindungselement 41 und dem zweiten Verbindungselement 42 eine im Wesentlichen vom Gelenk 24 vorgegebene Relativbewegung (z.B. scharnierartig) möglich ist.

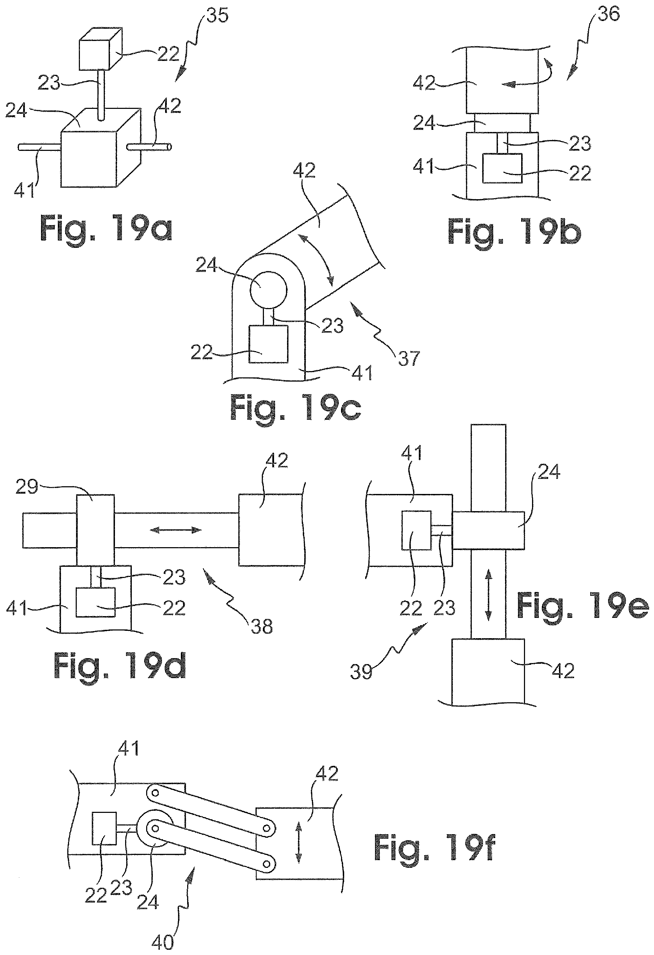

Es sind verschiedene Ausprägungen der Betätigungsvorrichtung 35 bekannt, von denen beispielhaft im Folgenden fünf verschiedene Ausprägungen der Betätigungsvorrichtung 35 beschrieben werden:
- eine Betätigungsvorrichtung 36 zur Rotation um eine vertikale Achse (siehe Fig. 19b),
- eine Betätigungsvorrichtung 37 zur Rotation/Verschwenkung um eine horizontale Achse (siehe Fig. 19c),
- eine Betätigungsvorrichtung 38 zur Translation entlang einer horizontalen Achse (siehe Fig. 19d),
- eine Betätigungsvorrichtung 39 zur Translation entlang einer vertikalen Achse (siehe Fig. 19e), sowie
- eine Betätigungsvorrichtung 40 zur Parallelverschiebung vornehmlich in vertikaler Richtung (siehe Fig. 19f).

Die Betätigungsvorrichtung 36 zur Rotation um eine vertikale Achse ist beispielhaft in Figur 19b dargestellt. Hierbei handelt es sich prinzipiell um einen Rotationsantrieb, beispielsweise einen Elektromotor oder ein Getriebe zur Transformation einer Translationsbewegung in eine Rotationsbewegung wie beispielsweise eine Kombination aus Zahnstange und Zahnrad. Diese Vorrichtung wird u.a. als Aktor zwischen der Basis und dem Rotationsarm der Positionsvorrichtung gemäß der Fig. 1 bis 5 eingesetzt.

Die Betätigungsvorrichtung 37 zur Rotation/Verschwenkung um eine horizontale Achse ist beispielhaft in Figur 19c dargestellt und kann entweder einen Rotationsantrieb gemäß vorstehender Definition aber auch einen Translationsantrieb wie eine Kolben-Zylinder-Einheit umfassen, der in einem Mittenabschnitt der gekoppelten Verbindungselemente angreift, die über ein Scharnier miteinander verbunden sind.

Die Betätigungsvorrichtung 38 zur Translation entlang einer horizontalen Achse ist beispielhaft in Figur 19d dargestellt und kann einen allgemein bekannten Teleskopmechanismus, eine Zahnstange oder eine Kolben-Zylinder-Einheit betreffen.

Die Betätigungsvorrichtung 39 zur Translation entlang einer vertikalen Achse ist beispielhaft in Figur 19e dargestellt und hat einen zur Fig. 19d vergleichbaren konstruktiven Aufbau. Vorteilhaft hierbei ist jedoch, dass die Betätigungsvorrichtung 39 beispielsweise durch Parallelschaltung einer Feder Kräftebalanciert ist, sodass auf den Antrieb zur Betätigung z.B. der Zahnstange/Teleskopstange im Wesentlichen nur Trägheitsmomente (und ggf. Reibungskräfte) einwirken.

Die Betätigungsvorrichtung 40 zur Parallelverschiebung vornehmlich in vertikaler Richtung ist beispielhaft in Figur 13f dargestellt. In diesem Fall wird diese durch ein Parallelscharnier gebildet bestehend aus mindestens zwei parallelen Hebeln, die jeweils endseitig mit einem Verbindungselement scharnierartig gekoppelt sind. Wenigstens einer der Hebel wird nach dem Prinzip der Betätigungsvorrichtung 37 angetrieben.

Das Funktionsprinzip der vorstehenden Betätigungsvorrichtungen lässt sich wie Folgt zusammenfassen:
Bei den Betätigungsvorrichtungen 36 und 38 ist die Betätigung durch den Aktuator unabhängig von der Kraft durch das Eigengewicht. Bei den Betätigungsvorrichtungen 37, 39 und 40 ist die Betätigung durch die Kraft durch das Eigengewicht beeinflusst. Der Aktuator 22 muss daher zusätzlich zur Betätigungskraft eine Kraft erzeugen, die der Kraft durch das Eigengewicht entgegengesetzt ist. Diese Kraft kann abhängig von der Position der Betätigungsvorrichtung sein (wie im Fall der Betätigungsvorrichtung 37 und 40), oder unabhängig d.h. konstant (wie im Fall der Betätigungsvorrichtung 39).

Die Notwendigkeit der Vorhaltung einer über die eigentliche Betätigungskraft hinausgehende Kraft durch den Aktuator 22 führt zu der Notwendigkeit, dies bei der Auslegung des Aktuators 22 entsprechend vorzusehen, wie dies in der Tabelle gemäß der Fig. 6 dargestellt wird. Folglich ist es erforderlich, dass der Aktuator 22 eine höhere Kraft erzeugen kann, als für die eigentliche Betätigung erforderlich. Beispielsweise bei Fehlfunktion der Überwachungsvorrichtung 31 und der Kraftbegrenzungsvorrichtung 32 oder bei Fehlbedienung besteht daher das Risiko, dass die vom Aktuator 22 vorgehaltene Kraft auf den Patientenkörper einwirkt und zu den Patienten gefährdenden Einwirkungen auf den Patientenkörper führt.

Die erfindungsgemäße Lösung ist die Implementierung einer Kraftkompensation, vorzugsweise durch eine konstruktive Vorrichtung (Vorspannfeder / Ausgleichsgewichte über Hebelmechanismen). Dadurch kann die Auslegung des Aktuators 22 derart gestaltet werden, dass der Aktuator 22 keine Kraft erzeugen kann, die über die zur Betätigung der Betätigungsvorrichtung 35 erforderliche Kraft hinausgeht. Vorzugsweise ist die zur Betätigung der Betätigungsvorrichtung 35 erforderliche Kraft nicht höher als die Kraft, die maximal auf den Patientenkörper einwirken darf, ohne eine den Patienten gefährdende Einwirkung auf den Patientenkörper zu erzeugen. Durch diese Maßnahme ist das Risiko quasi ausgeschlossen, dass selbst bei einer Fehlfunktion der Überwachungsvorrichtung 31 oder der Kraftbegrenzungsvorrichtung 32 gemäß der vorstehenden Beschreibung oder bei einer Fehlbedienung der magnetischen Führungseinrichtung (Robotik) bestehend aus Positionsvorrichtung und Endeffektor die auf den Patientenkörper einwirkende Kraft zu den Patienten gefährdenden Einwirkungen führt.

### Bezugszeichenliste

Freiheitsgrad a: Translatorische Bewegung des Magnetfelderzeugers kompakter Bauweise entlang der Raumachse x
Freiheitsgrad b: Translatorische Bewegung des Magnetfelderzeugers kompakter Bauweise entlang der Raumachse y
Freiheitsgrad c: Translatorische Bewegung des Magnetfelderzeugers kompakter Bauweise entlang der Raumachse z
Freiheitsgrad d: Rotatorische Bewegung des Magnetfelderzeugers kompakter Bauweise um die interne Achse A in 0-Lage (bzw. um die Y-Achse des Raum-Koordiantensystems unabhängig von der Magnetfelderzeuger-Lage)
Freiheitsgrad e: Rotatorische Bewegung des Magnetfelderzeugers kompakter Bauweise um die interne Achse B
   - 1: Magnetfelderzeuger kompakter Bauart
   - 2: Raumachse x des Magnetfelderzeugers kompakter Bauart
   - 3: Raumachse y des Magnetfelderzeugers kompakter Bauart
   - 4: Raumachse z des Magnetfelderzeugers kompakter Bauart
   - 5: Raumachse a des Magnetfelderzeugers kompakter Bauart
   - 6: Raumachse b des Magnetfelderzeugers kompakter Bauart
   - 7: Polarisationsachse des Magnetfelderzeugers kompakter Bauart
   - 8: Aktuator zur Aktuierung eines rotatorischen Freiheitsgrades
   - 9: Übertragungselement zur Aktuierung eines rotatorischen Freiheitsgrades
   - 10: Sensorelement
   - 11: Bedienelement
   - 12: Gehäuse des magnetischen Endeffektors
   - 13: Magnetischer Endeffektor
   - 14: Verbindungselement zur Übertragung von Kräften
   - 15: Verbindungselement zur Übertragung von Energie
   - 16: Verbindungselement zur Übertragung von Daten
   - 17: Sensorelement
   - 18: Sensorelement
   - 19: Gehäuse der Positioniervorrichtung
   - 20: Bedienelement
   - 21: Positioniervorrichtung
   - 22: Aktuator
   - 23: Übertragungselement
   - 24: Gelenk
   - 25: Mechanisches Verbindungselement
   - 26: Verbindungselement zur Übertragung von Kräften
   - 27: Befestigungselement
   - 28: Regelungseinrichtung
   - 29: Übertragungselement für Daten
   - 30: Übertragungselement für Daten
   - 31: Überwachungsvorrichtung
   - 32: Kraftbegrenzungsvorrichtung
   - 33: Erstes Element der Übertragungsvorrichtung
   - 34: Zweites Element der Übertragungsvorrichtung
   - 35: Betätigungsvorrichtung
   - 36: Betätigungsvorrichtung zur Rotation um eine vertikale Achse
   - 37: Betätigungsvorrichtung zur Rotation um eine horizontale Achse
   - 38: Betätigungsvorrichtung zur Translation entlang einer horizontalen Achse
   - 39: Betätigungsvorrichtung zur Translation entlang einer vertikalen Achse
   - 40: Betätigungsvorrichtung zur Parallelverschiebung vornehmlich in vertikaler Richtung
   - 41: Erstes Verbindungselement
   - 42: Zweites Verbindungselement
   - 100: Führungseinrichtung
   - 102: Positioniervorrichtung
   - 104: Effektor (Gehäuse)
   - 106: Anschlussinterface
   - 108/110: Schwenkarme
   - 112/116: Scharniergelenk
   - 114/122/124/128: Aktor
   - 118: Rotationsarm
   - 120: horizontales Scharnier
   - 126: Basis
   - 132: Auszug/Teleskopstange
   - 130/134: Aktuator
   - 136: vertikales Scharnier
   - 138: Schubstange
   - 140: Hebelarm
   - 142: Gewicht
   - 144: Zug-/Druckstange
   - 146: Hebelstange
   - 148: Zugstange
   - 150: (Ausgleichs-) Gewicht

## Patentansprüche

1. Extrakorporale Führungseinrichtung für ein intrakorporales magnetisches Objekt mit einer motorisch angetriebenen Positioniervorrichtung (102), die maximal drei zu aktivierende Freiheitsgrade nämlich zur translatorischen Bewegung eines distalen Anschlussinterface (106) der Positioniervorrichtung (102) in einem extrakoporalen Raum-Koordinatensystem hat, an das ein Endeffektor (104) der extrakorporalen Führungseinrichtung angeschlossen ist, der maximal zwei zu aktivierende Freiheitsgrade nämlich zur rotatorischen Bewegung eines Magnetfelderzeugers (1) vorzugsweise Permanentmagneten des Endeffektors (104) hat, **dadurch gekennzeichnet, dass** zumindest einer der zwei Freiheitsgrade des Magnetfelderzeugers (1) in einem Effektorgehäuse (104a) eingehaust ist.

2. Extrakorporale Führungseinrichtung nach Anspruch 1, **dadurch gekennzeichnet, dass** das Effektorgehäuse (104a) entweder aus im Wesentlichen geschlossenen Gehäusewänden und/oder aus einer offenen Gitter-/Rahmenstruktur besteht.

3. Extrakorporale Führungseinrichtung nach Anspruch 1 oder 2, **dadurch gekennzeichnet, dass** die drei Freiheitsgrade der Positioniervorrichtung (102) ausschließlich eine translatorische Bewegung des Anschlussinterface (106) in eine X-, Y- und Z-Richtung des Raum-Koordinatensystems erlauben, in welchem die Y-Achse vorzugsweise im Wesentlichen längs der Schwerkraftrichtung und die Z-Achse entlang der Längs- und damit der Polarisationsachse des Magnetfelderzeugers (1) im Fall seiner horizontalen Raumlage als Ausgangs- oder 0-Lage ausgerichtet ist, wohingegen die zwei Freiheitsgrade des Magnetfelderzeugers (1) ausschließlich jeweils eine Rotationsbewegung des Magnetfelderzeugers (1), um jeweils eine Achse, vorzugsweise die Y- und X-Achse des Raum-Koordinatensystems erlauben, um ein Gieren und Nicken des Magnetfelderzeugers (1) bezüglich des Raum-Koordinatensystems zu bewirken.

4. Extrakorporale Führungseinrichtung nach Anspruch 1 oder 2, **dadurch gekennzeichnet, dass** die drei Freiheitsgrade der Positioniervorrichtung (102) eine translatorische Bewegung des Anschlussinterface (106) in eine X-, Y- und Z-Richtung des Raum-Koordinatensystems erlauben, in welchem die Y-Achse vorzugsweise im Wesentlichen längs der Schwerkraftrichtung ausgerichtet ist, wohingegen die zwei Freiheitsgrade des Magnetfelderzeugers (1) jeweils eine Rotationsbewegung des Magnetfelderzeugers (1), um ein Schwenken insbesondere im wesentlichen um die Vertikalachse und ein Nicken der Polarisationsachse des Magnetfelderzeugers (1) bezüglich des Raum-Koordinatensystems zu bewirken.

5. Extrakorporale Führungseinrichtung nach Anspruch 3 oder 4, **dadurch gekennzeichnet, dass** nur der eine rotatorische Freiheitsgrad für ein Nicken des Magnetfelderzeugers (1) und vorzugsweise für ein Rotieren des Magnetfelderzeugers (1) um die X-Achse eingehaust ist, wobei das Gehäuse (104a) oder zumindest ein Teil (104a) des Gehäuses (104a, 104b) drehbar für ein Gieren des darin gelagerten Magnetfelderzeugers (1) vorzugsweise für ein Rotieren um die Y-Achse am Anschlussinterface (106) gelagert ist.

6. Extrakorporale Führungseinrichtung nach Anspruch 3 oder 4, **dadurch gekennzeichnet, dass** beide Freiheitsgrade für ein Nicken und Gieren eingehaust sind, wohingegen das Gehäuse (104a) in seiner räumlichen Ausrichtung unabhängig von der Bewegung der Positioniervorrichtung (102) gleich bleibt.

7. Extrakorporale Führungseinrichtung nach einem der vorstehenden Ansprüche, **dadurch gekennzeichnet, dass** die Positioniervorrichtung (102) eine Anzahl von jeweils motorisch angetriebenen Auslegern oder Armen (108, 110) hat, von denen ausgewählte Ausleger oder Arme bezüglich der zugehörigen motorischen Antriebe (114, 122) gewichtsausbalanciert sind, wofür vorzugsweise ein solches Balanciersystem vorgesehen ist, das eine dynamische Anpassung der Gewichtsausbalancierung an die Gewichtsbelastung vornimmt oder bewirkt, welche aktuell auf den jeweiligen motorischen Antrieb einwirkt.

8. Extrakorporale Führungseinrichtung nach einem der vorstehenden Ansprüche, **dadurch gekennzeichnet, dass** die Positioniervorrichtung (102) eine Anzahl von jeweils motorisch angetriebenen Auslegern oder Armen hat, von denen ausgewählte Ausleger oder Arme (108, 110) bezüglich der zugehörigen motorischen Antriebe (114, 124), vorzugsweise nach dem Scararobotik-Prinzip, so gelagert sind, dass die motorischen Antriebe keine oder nur eine geringe statische Gewichtslast aufnehmen oder überwinden müssen, wofür die vorbestimmte Bewegungsrichtung der Ausleger vorzugsweise im Wesentlichen senkrecht zur Schwerkraft weist.

9. Extrakorporale Führungseinrichtung nach einem der vorstehenden Ansprüche, **dadurch gekennzeichnet, dass** der jeweilige motorische Antrieb für ausgewählte Freiheitsgrade antriebskraftbegrenzt ist oder wird, derart, dass die maximal abgebbare Antriebskraft gleich oder größer ist als eine zu erwartende Betriebs-Belastungskraft, jedoch gleich oder kleiner ist als eine vorbestimmte Maximalkraft, bei der eine Verletzung eines Patienten oder einer Bedienperson im Kollisionsfall weitgehend ausgeschlossen wird.

10. Extrakorporale Führungseinrichtung nach einem der vorstehenden Ansprüche, **dadurch gekennzeichnet, dass** der jeweilige motorische Antrieb für ausgewählte Freiheitsgrade mittels einer nachgeschalteten Rutsch- oder Sicherheitskupplung, eines vorgeschalteten elektrischen Strom- oder Hydraulik-/Pneumatikdruckbegrenzers oder durch Verwendung eines Schrittmotors antriebskraftbegrenzt ist.

11. Extrakorporale Führungseinrichtung nach einem der vorstehenden Ansprüche, **dadurch gekennzeichnet, dass** der jeweilige motorische Antrieb einen Elektromotor, ein Piezoelement, eine hydraulische/pneumatische KolbenZylindereinheit oder einen Magnetantrieb hat.

12. Extrakorporale Führungseinrichtung nach einem der vorstehenden Ansprüche, **gekennzeichnet durch** eine Überwachungseinrichtung, an die eine Anzahl von Sensoren angeschlossen ist zur Überwachung des Betriebszustands der Führungseinrichtung und zur Korrektur des Betriebszustands und/oder Abschaltung der Führungseinrichtung bei erfasstem vorbestimmten Unfallrisiko.

13. Extrakorporale Führungseinrichtung nach Anspruch 12, **dadurch gekennzeichnet, dass** die Sensoren ausgewählt sind aus einer Gruppe von Sensoren umfassend:
- ein Kraftsensor zur Erfassung der Antriebslast,
- ein Berührungs- oder Kraftsensor zur Erfassung der Kontaktkraft zwischen dem magnetischen Endeffektor oder einem ausgewählten Ausleger der Positioniervorrichtung und einer Bedienperson oder einem Patienten,
- ein Deformations-Erfassungssensor zur Erfassung einer Eindellung vorzugsweise am Endeffektorgehäuse,
- ein optischer Sensor zur optischen Erfassung von Hindernissen und
- ein Abstandssensor zur Erfassung eines Abstands zu Hindernissen.

14. Extrakorporale Führungseinrichtung nach einem der vorstehenden Ansprüche, **gekennzeichnet durch** Bewegungsbegrenzungsmittel vorzugsweise in Form von programmierten/programmierbaren und/oder mechanischen Endanschlägen/Endschaltern, welche so an der Positioniervorrichtung und/oder dem magnetischen Endeffektor montiert sind, dass eine Auswahl der maximal drei Freiheitsgrade der Positioniervorrichtung und/oder der maximal zwei Freiheitsgrade des Magnetfelderzeugers auf einen vorbestimmten Bewegungsbereich mechanisch eingeschränkt ist.

15. Extrakorporale Führungseinrichtung nach einem der vorstehenden Ansprüche **dadurch gekennzeichnet, dass** das Gehäuse (104a) des Endeffektors (104) derart am Ausgangs-/Anschussinterface (106) der Positionsvorrichtung (102) angeschlossen ist, dass es mit oder ohne eigenen motorischen Antrieb sowie unabhängig von der aktuellen Bewegung der Positioniervorrichtung (102) und/oder des Magnetfelderzeugers (1) grundsätzlich im Wesentlichen in Schwerkraftrichtung ausgerichtet ist, derart, dass eine vorgesehene Kontaktfläche am Gehäuse (104a) immer die gleiche Ausrichtung zur Schwerkraftrichtung behält.

## Claims

1. An extracorporeal guiding device for an intracorporeal magnetic object comprising a motor-driven positioning device (102) having a maximum of three degrees of freedom to be activated namely for translational motion of a distal connecting interface (106) of the positioning device (102) in an extracorporeal space coordinate system to which an end effector (104) of the extracorporeal guiding device is connected, the end effector having a maximum of two degrees of freedom to be activated namely for rotational motion of a magnetic field generator (1), preferably a permanent magnet of the end effector (104), **characterized in that** at least one of the two degrees of freedom of the magnetic field generator (1) is encased in an effector housing (104a).

2. The extracorporeal guiding device according to claim 1, **characterized in that** the effector housing (104a) consists either of substantially closed housing walls and/or of an open grid/frame structure.

3. The extracorporeal guiding device according to claim 1 or 2, **characterized in that** the three degrees of freedom of the positioning device (102) exclusively allow a translational motion of the connecting interface (106) in an X-, Y- and Z-direction of the space coordinate system in which the Y-axis is preferably orientated substantially along the gravity direction and the Z-axis is orientated along the longitudinal and thus polarization axis of the magnetic field generator (1) in the case of its horizontal space position as home or 0-position, whereas the two degrees of freedom of the magnetic field generator (1) exclusively allow a respective rotational motion of the magnetic field generator (1) about a respective axis, preferably the Y- and X-axis of the space coordinate system so as to cause yawing and pitching of the magnetic field generator (1) with respect to the space coordinate system.

4. The extracorporeal guiding device according to claim 1 or 2, **characterized in that** the three degrees of freedom of the positioning device (102) allow a translational motion of the connecting interface (106) in an X-, Y- and Z-direction of the space coordinate system in which the Y-axis is preferably orientated substantially along the gravity direction, whereas each of the two degrees of freedom of the magnetic field generator (1) allows a rotational motion of the magnetic field generator (1) so as to cause pivoting especially substantially about the vertical axis and pitching of the polarization axis of the magnetic field generator (1) with respect to the space coordinate system.

5. The extracorporeal guiding device according to claim 3 or 4, **characterized in that** only the one rotational degree of freedom is encased for pitching of the magnetic field generator (1) and preferably for rotating of the magnetic field generator (1) about the X-axis, the housing (104a) or at least part (104a) of the housing (104a, 104b) is pivot-mounted for yawing of the magnetic field generator (1) supported therein preferably for rotating about the Y-axis at the connecting interface (106).

6. The extracorporeal guiding device according to claim 3 or 4, **characterized in that** both degrees of freedom are encased for pitching and yawing, whereas the housing (104a) is maintained in its spatial orientation independently of the motion of the positioning device (102).

7. The extracorporeal guiding device according to one of the preceding claims, **characterized in that** the positioning device (102) includes a number of motor-driven extensions or arms (108, 110), wherein selected extensions or arms thereof are weight-balanced with respect to the corresponding motor drives (114, 124), for which purpose preferably such a balancing system is provided which performs or causes a dynamic adaptation of the weight balancing to the weight load currently acting on the respective motor drive.

8. The extracorporeal guiding device according to one of the preceding claims, **characterized in that** the positioning device (102) includes a number of motor-driven extensions or arms, wherein selected extensions or arms (108, 110) thereof are supported with respect to the corresponding motor drives (114, 124), preferably according to the SCARA robotics principle, so that the motor drives have to absorb or overcome no or only a small static weight load, for which purpose the predetermined direction of movement of the extensions preferably points substantially normal to gravity.

9. The extracorporeal guiding device according to one of the preceding claims, **characterized in that** the respective motor drive for selected degrees of freedom is or becomes limited as to the driving force in such manner that the driving force adapted to be maximally output is equal to or greater than an operating load force to be expected, but equal to or smaller than a predetermined maximum force in the case of which an injury of a patient or an operator in the event of collision is largely excluded.

10. The extracorporeal guiding device according to one of the preceding claims, **characterized in that** the respective motor drive for selected degrees of freedom is limited as to the driving force by means of a downstream connected safety-friction clutch or safety clutch, an upstream electric power or hydraulic/pneumatic pressure limiter or by use of a step motor.

11. The extracorporeal guiding device according to one of the preceding claims, **characterized in that** the respective motor drive includes an electric motor, a piezo element, a hydraulic/pneumatic piston-cylinder unit or a magnetic drive.

12. The extracorporeal guiding device according to one of the preceding claims, **characterized by** a monitoring means to which a number of sensors is connected for monitoring the operating state of the guiding device and for correcting the operating state and/or turning off the guiding device when a predetermined risk of accident is detected.

13. The extracorporeal guiding device according to claim 12, **characterized in that** the sensors are selected from a group of sensors comprising:
- a force sensor for detecting the drive load,
- a touch or force sensor for detecting the contact force between the magnetic end effector or a selected extension of the positioning device and an operator or a patient,
- a deformation detection sensor for detecting an indentation preferably at the end effector housing,
- an optical sensor for optically detecting obstacles and
- a distance sensor for detecting a distance from obstacles.

14. The extracorporeal guiding device according to one of the preceding claims, **characterized by** motion limiting means preferably in the form of programmed/programmable and/or mechanical limit stops/limit switches mounted to the positioning device and/or the magnetic end effector such that a selection of the maximum of three degrees of freedom of the positioning device and/or the maximum of two degrees of freedom of the magnetic field generator is mechanically restricted to a predetermined range of motion.

15. The extracorporeal guiding device according to one of the preceding claims, **characterized in that** the housing (104a) of the end effector (104) is connected to the output/connecting interface (106) of the positioning device (102) such that it is basically orientated substantially in the gravity direction with or without an inherent motor drive as well as independently of the current motion of the positioning device (102) and/or of the magnetic field generator (1) in such manner that a provided contact face at the housing (104a) always maintains the same orientation with respect to the gravity direction.

## Revendications

1. Dispositif de guidage extracorporel pour un objet magnétique intracorporel avec un dispositif de positionnement à commande motorisée (102), qui a au maximum trois degrés de liberté à activer à savoir pour le déplacement en translation d'une interface de connexion distale (106) du dispositif de positionnement (102) dans un système extracorporel de coordonnées spatiales, auquel est raccordé un effecteur terminal (104) du dispositif de guidage extracorporel, qui a au maximum deux degrés de liberté à activer à savoir pour le déplacement en rotation d'un générateur de champ magnétique (1) de préférence des aimants permanents de l'effecteur terminal (104), **caractérisé en ce qu'**au moins l'un des deux degrés de liberté du générateur de champ magnétique (1) est logé dans un boîtier d'effecteur (104a).

2. Dispositif de guidage extracorporel selon la revendication 1, **caractérisé en ce que** le boîtier d'effecteur (104a) est constitué de parois de boîtier sensiblement fermées et/ou d'une structure de châssis/treillis.

3. Dispositif de guidage extracorporel selon la revendication 1 ou 2, **caractérisé en ce que** les trois degrés de liberté du dispositif de positionnement (102) permettant exclusivement un déplacement en translation de l'interface de connexion (106) dans une direction X, Y et Z du système de coordonnées spatiales, dans lequel l'axe Y est orienté de préférence sensiblement dans le sens de la pesanteur et l'axe Z le long de l'axe longitudinal et donc de l'axe de polarisation du générateur de champ magnétique (1) dans le cas de sa position spatiale horizontale comme position de sortie ou position 0, tandis que les deux degrés de liberté du générateur de champ magnétique (1) permettent exclusivement respectivement un déplacement rotatif du générateur de champ magnétique (1) autour d'un axe respectif, de préférence des axes Y et X du système de coordonnées spatiales pour provoquer un mouvement de lacet et de tangage du générateur de champ magnétique (1) par rapport au système de coordonnées spatiales.

4. Dispositif de guidage extracorporel selon la revendication 1 ou 2, **caractérisé en ce que** les trois degrés de liberté du dispositif de positionnement (102) permettent un déplacement en translation de l'interface de connexion (106) dans une direction X, Y et Z du système de coordonnées spatiales, dans lequel l'axe Y est orienté de préférence sensiblement dans le sens de la pesanteur, tandis que les deux degrés de liberté du générateur de champ magnétique (1) permettent respectivement un mouvement de rotation du générateur de champ magnétique (1) pour provoquer un pivotement en particulier en substance autour de l'axe vertical et un mouvement de tangage de l'axe de polarisation du générateur de champ magnétique (1) par rapport au système de coordonnées spatiales.

5. Dispositif de guidage extracorporel selon la revendication 3 ou 4, **caractérisé en ce que** seul le premier degré de liberté en rotation est logé pour un mouvement de tangage du générateur de champ magnétique (1) et de préférence pour une rotation du générateur de champ magnétique (1) autour de l'axe X, dans lequel le boîtier (104a) ou au moins une partie (104a) du boîtier (104a, 104b) est monté(e) à rotation pour un mouvement de lacet du générateur de champ magnétique (1) qui y est monté, de préférence pour une rotation autour de l'axe Y sur l'interface de connexion (106).

6. Dispositif de guidage extracorporel selon la revendication 3 ou 4, **caractérisé en ce que** les deux degrés de liberté sont logés pour un mouvement de tangage et de lacet, tandis que le boîtier (104a) reste égal dans son orientation spatiale indépendamment du mouvement du dispositif de positionnement (102).

7. Dispositif de guidage extracorporel selon l'une quelconque des revendications précédentes, **caractérisé en ce que** le dispositif de positionnement (102) a un nombre d'empattements ou de bras respectivement à commande motorisée (108, 110) parmi lesquels des empattements ou des bras choisis sont équilibrés en poids par rapport aux commandes motorisées associées (114, 122), pour lequel il est prévu de préférence un système d'équilibrage qui effectue ou induit une adaptation dynamique de l'équilibrage de poids à la sollicitation pondérale qui agit couramment sur la commande motorisée respective.

8. Dispositif de guidage extracorporel selon l'une quelconque des revendications précédentes, **caractérisé en ce que** le dispositif de positionnement (102) a un nombre d'empattements ou de bras respectivement à commande numérique, dont des empattements ou des bras choisis (108, 110) sont montés par rapport aux commandes motorisées associées (114, 124) de préférence selon le principe de la robotique Scara en sorte que les commandes motorisées ne doivent pas recevoir ou surmonter une charge pondérale statique ou seulement une faible charge pondérale, si bien que le sens de déplacement prédéterminé des empattements se présente de préférence sensiblement perpendiculaire à la pesanteur.

9. Dispositif de guidage extracorporel selon l'une quelconque des revendications précédentes, **caractérisé en ce que** la commande motorisée respective pour les degrés de liberté choisis a été ou est limitée en force de commande de sorte que la force de commande délivrable au maximum soit égale ou supérieure à une force de sollicitation de commande à attendre, mais soit égale ou plus petite qu'une force maximale prédéterminée, dans laquelle une blessure d'un patient ou d'une personne de service est largement exclue dans le cas d'une collision.

10. Dispositif de guidage extracorporel selon l'une quelconque des revendications précédentes, **caractérisé en ce que** la commande motorisée respective pour les degrés de liberté choisis est limitée en force de commande au moyen d'un couplage à friction connecté en aval, d'un limiteur de courant électrique ou de pression hydraulique/pneumatique connecté en amont ou par utilisation d'un moteur pas-à-pas.

11. Dispositif de guidage extracorporel selon l'une quelconque des revendications précédentes, **caractérisé en ce que** la commande motorisée respective a un moteur électrique, un élément piézoélectrique, une unité piston-cylindre hydraulique/pneumatique ou une commande magnétique.

12. Dispositif de guidage extracorporel selon l'une quelconque des revendications précédentes, **caractérisé par** un dispositif de surveillance auquel un certain nombre de capteurs est raccordé pour surveiller l'état de fonctionnement du dispositif de guidage et pour corriger l'état de fonctionnement et/ou la déconnexion du dispositif de guidage dans le cas d'un risque d'accident prédéterminé détecté.

13. Dispositif de guidage extracorporel selon la revendication 12, **caractérisé en ce que** les capteurs sont choisis dans un groupe de capteurs comprenant :
- un capteur de force pour détecter la charge de commande,
- un capteur de contact ou de force pour détecter la force de contact entre l'effecteur terminal magnétique ou un empattement choisi du dispositif de positionnement et une personne de service ou un patient,
- un capteur de détection de déformation pour détecter une indentation de préférence sur le boîtier de l'effecteur terminal ;
- un capteur optique pour la détection optique d'obstacles et
- un capteur de distance pour détecter une distance vis-à-vis d'obstacles.

14. Dispositif de guidage extracorporel selon l'une quelconque des revendications précédentes, **caractérisé par** des moyens de limitation de déplacement de préférence sous la forme de butées ou de commutateurs de fin de course programmés/programmables et/ou mécaniques, qui sont montés sur le dispositif de positionnement et/ou l'effecteur terminal magnétique de sorte qu'un choix des trois degrés de liberté au maximum du dispositif de positionnement et/ou des deux degrés de liberté au maximum du générateur de champ magnétique soit limité mécaniquement dans une plage de déplacement prédéterminée.

15. Dispositif de guidage extracorporel selon l'une quelconque des revendications précédentes, **caractérisé en ce que** le boîtier (104a) de l'effecteur terminal (104) est connecté à l'interface de sortie/connexion (106) du dispositif de positionnement (102) de sorte qu'il soit orienté avec ou sans commande motorisée propre ainsi qu'indépendamment du déplacement courant du dispositif de positionnement (102) et/ou du générateur de champ magnétique (1) en principe en substance dans le sens de la pesanteur de sorte qu'une surface de contact prévue sur le boîtier (104a) conserve toujours la même orientation dans le sens de la pesanteur.
